Europäisches Patentamt

European Patent Office

Office européen des brevets

(19)

(11) **EP 1 543 765 A1**

(12) **EUROPEAN PATENT APPLICATION**
published in accordance with Art. 158(3) EPC

(43) Date of publication:
**22.06.2005 Bulletin 2005/25**

(51) Int Cl.⁷: **A61B 1/00**

(21) Application number: **03795259.5**

(22) Date of filing: **29.08.2003**

(86) International application number:
**PCT/JP2003/011081**

(87) International publication number:
**WO 2004/023986 (25.03.2004 Gazette 2004/13)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **30.08.2002 JP 2002255696**
**30.08.2002 JP 2002255700**

(71) Applicant: **Olympus Corporation**
**Tokyo 151-0072 (JP)**

(72) Inventors:
 • **ONISHI, Junichi**
 **Hachioji-shi, Tokyo 193-0823 (JP)**

 • **AKIMOTO, Shunya**
 **Hachioji-shi, Tokyo 192-0032 (JP)**
 • **KAJI, Kunihide**
 **Hachioji-shi, Tokyo 192-0023 (JP)**
 • **KOBAYASHI, Eiichi**
 **Tama-shi, Tokyo 206-0025 (JP)**
 • **SAITO, Akito**
 **Hino-shi, Tokyo 191-0001 (JP)**
 • **SHIBASAKI, Takao**
 **Meguro-ku, Tokyo 153-0043 (JP)**
 • **MINE, Taiji**
 **Chofu-shi, Tokyo 182-0007 (JP)**

(74) Representative: **von Hellfeld, Axel, Dr.**
 **Schweigerstrasse 2**
 **81541 München (DE)**

(54) **MEDICAL TREATMENT SYSTEM, ENDOSCOPE SYSTEM, ENDOSCOPE INSERT OPERATION PROGRAM, AND ENDOSCOPE DEVICE**

(57)     An analyzing unit 9 is connected to an inserting-operation information collecting unit 5, an endoscope video output unit 6, and a processing device 2, and receives live inserting-operation information and endoscope image, standard inserting-operation information as stored information, and an endoscope image correlated therewith (endoscope image which is obtained by picking up an image of a sample at the position of a distal-end portion of an inserting portion detected based on the inserting-operation information). The analyzing unit 9 collects in real time the inserting-operation information and the endoscope image from the inserting-operation information collecting unit 5 and the endoscope video output unit 6 under the bronchoscope operation of an operator. Further, the analyzing unit 9 sequentially analyzes the collected inserting-operation information and endoscope image by comparing with the standard inserting-operation information and endoscope image correlated with the inserting-operation information, which are stored in a storing unit 2A of the processing device 2, outputs an analysis result to a notifying unit 10, and monitors the inserting operation of the endoscope inserting portion performed by the operator.

FIG.2

**Description**

Technical Field

The present invention relates to a medical treatment system, an endoscope system, an endoscope inserting-operation program, and an endoscope apparatus which can perform the inserting operation of an inserting portion of, e.g., an endoscope to the lumen in the body cavity.

Background Art

Recently, an endoscope system and an endoscope apparatus have widely been used. In the endoscope system and the endoscope apparatus, a long inserting portion is inserted in the body cavity to observe the organs in the body cavity, and various cure and treatments are performed by using a treatment tool which is inserted in a channel for inserting the treatment tool if needed.

Further, recently, in the medical endoscope system and endoscope apparatus, a tomographic image of a sample is picked by a CT (Computed Tomography) and the diagnosis of the diseased part is performed by a three-dimensional image which is obtained from the tomographic image.

One of the three-dimensional images is that of the lung bronchi. The three-dimensional image of the lung bronchi is used for three-dimensionally grasping the position of an abnormal portion which is suspected to be lung cancer or the like. For the purpose of biopsy of the abnormal portion which is found as mentioned above, the diagnosis is performed by sampling the tissues of the abnormal part with a biopsy projected from the distal-end portion of an inserting portion of a bronchoscope, which is inserted in the lung bronchi.

Generally, the endoscope comprises, at the inserting portion which is inserted in the lumen or at the distal-end portion thereof, observing means such as an objective optical system and an image pick-up device for obtaining an observed image of the lumen, or an objective optical system and an image guide fiber. Further, the endoscope has, on the distal-end portion side of the inserting portion, a bending portion for freely directing the distal-end portion. In the endoscope with the above-mentioned structure, the distal-end portion of the inserting portion is positioned in various desired directions by bending the bending portion, or by turning the inserting portion and then inserting it in the complicated-shaped lumen.

In this case, in the lumen like the bronchi in the body cavity having many branches, when the abnormal portion is close to the end of the branch, the inserting portion of the endoscope does not accurately reach the target portion for a short time.

In order to solve the problems, Japanese Unexamined Patent Application Publication No. 2000-135215 suggests a device which guides a bronchoscope to a target portion by forming the three-dimensional image of the lumen in the sample based on three-dimensional image data of the sample, obtaining the route reaching the target point along the lumen on the three-dimensional image, forming a virtual endoscope image of the lumen along the route based on the image data, and displaying the virtual endoscope image on a monitor.

In the above-mentioned conventional endoscope apparatus, the monitor displays a live endoscope image of the sample picked up by the bronchoscope and also the virtual endoscope image of the bronchi, thereby guiding the inserting destination of the endoscope inserting portion. In this case, an operator inserts the endoscope inserting portion in the bronchi by properly rotating the endoscope inserting portion or by bending the bending portion while viewing the three-dimensional virtual endoscope image and the live endoscope image.

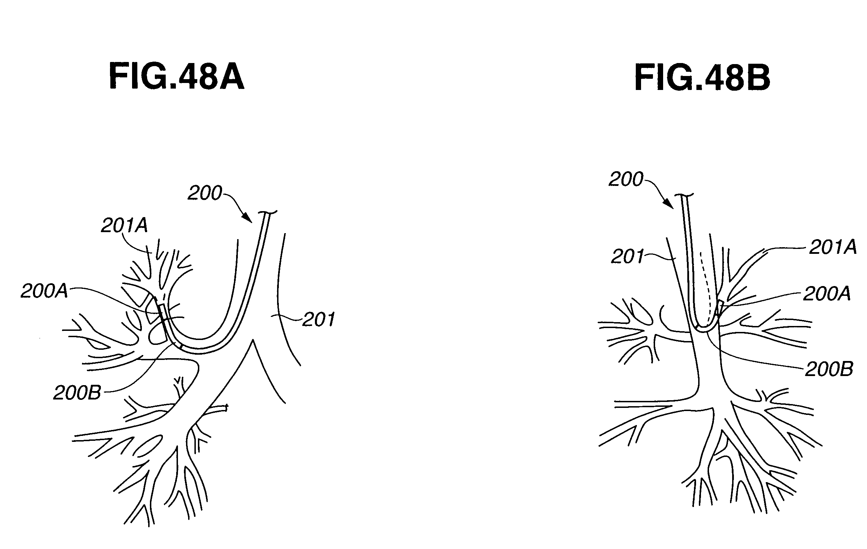

However, the bronchi has many branches and the images at the branches become similar images having a plurality of branching routes. For example, referring to Figs. 48A and 48B, in a bronchoscope 200, a bending portion 200B is bent so that a distal-end portion 200A of the inserting-portion reaches the interest point in a peripheral portion 201A of a branched bronchi 201.

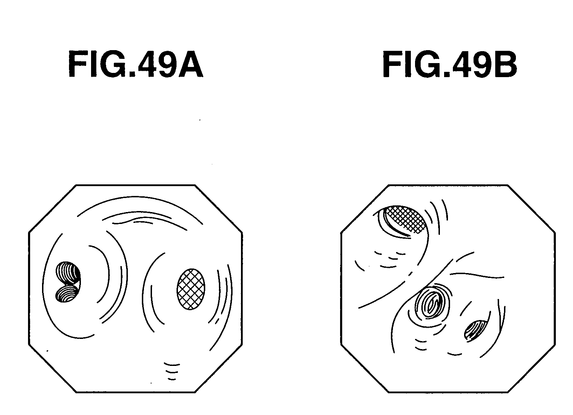

Referring to Fig. 49B, in the bronchoscope, when the obtained endoscope image has a characteristic branching structure, the branching direction of the bronchus having the interest portion is easily distinguished. However, referring to Fig. 49A, in the bronchoscope, it is not characteristic in the right and left branches and therefore when the gravity direction is not determined, the branching direction having the interest portion is not distinguished only from the image.

Further, Japanese Unexamined Patent Application Publication No. 5-127100 and U.S. Patent Publication No. 5,280,781 disclose a gravity direction instructing apparatus for endoscope which can check a relationship between an endoscope and the gravity direction. Japanese Unexamined Patent Application Publication No. 11-281897 which has been applied by the applicant of the present invention discloses an endoscope which can detect the gravity direction by providing a gravity detecting unit such as a gyroscope.

However, as disclosed in Japanese Unexamined Patent Application Publication No. 11-281897, the gravity detecting means such as the gyroscope is not provided on the distal-end portion of the inserting portion in the bronchoscope which has the limitation at the outer diameter of the inserting portion in the endoscope.

Thus, in the conventional bronchoscope apparatus, the gravity direction of the distal-end portion of the inserting portion is not accurately detected. The branching direction of the bronchus having the interest portion is not specified. Actually, by a skilled doctor having rich experiences of the endoscope operation, the distal-end portion of the bronchoscope precisely reaches the target portion for a short time.

It is an object of the present invention to provide an endoscope system, an endoscope inserting-operating program, and an endoscope apparatus, in which an inserting portion of an endoscope is accurately inserted to the target portion in the body cavity without fail for a short time.

Disclosure of Invention

According to the present invention, a medical treatment system having a long inserting portion which is inserted in a sample, comprises: a positional relationship detecting unit which detects a relative positional relationship between the sample and a distal-end portion of the inserting portion; an information input unit which can input predetermined information; and a storing unit which stores the predetermined information and the positional information detected by the positional relationship detecting unit with a correlation therebetween.

Further, according to the present invention, an endoscope system having an inserting portion which is inserted in a sample, comprises: a positional relationship detecting unit which detects a relative positional relationship between the sample and a distal-end portion of the inserting portion; an information input unit which can input predetermined information; and a storing unit which stores the predetermined information and the positional information detected by the positional relationship detecting unit with a correlation therebetween.

Furthermore, according to the present invention, an endoscope inserting-operation program for inserting an endoscope inserting portion in a sample, comprises: a positional relationship detecting step for detecting a relative positional relationship between the sample and a distal-end portion of the inserting portion; an information input step for inputting predetermined information; and a storing step for storing the predetermined information and the positional information detected by the positional relationship detecting step with a correlation therebetween.

In addition, according to the present invention, an endoscope system having an inserting portion for insertion in a sample, comprises: a storing unit which stores predetermined information which is previously correlated with relative positional information between the sample and a distal-end portion of the inserting portion; a positional relationship detecting unit which detects a relative positional relationship between the sample and the distal-end portion of the inserting portion; and an information output unit which can output, from the storing portion, predetermined information which is correlated with the positional relationship information detected by the positional relationship detecting unit.

In addition, according to the present invention, an endoscope inserting-operation program for inserting an endoscope inserting portion in a sample, comprises: a positional relationship detecting step for detecting a relative positional relationship between the sample and a distal-end portion of the inserting portion; and an information output step for outputting predetermined information corresponding to positional information detected by the positional relationship detecting step, from a storing unit which previously stores predetermined information that is correlated with the relative positional relationship between the sample and the distal-end portion of the inserting portion.

In addition, according to the present invention, an endoscope apparatus comprises: a detecting unit which detects inserting-operation information of an endoscope inserting portion which is inserted in a sample; a storing unit which stores standard inserting-operation information that is detected by the detecting unit and an endoscope image obtained by picking up an image of the sample taken upon the inserting operation at the position of a distal-end portion of the endoscope inserting portion with a correlation between; and a control unit which compares the standard inserting-operation information stored in the storing unit with the inserting-operation information obtained from the detecting unit during the operation and which monitors the inserting operation situation of the endoscope inserting portion.

In addition, according to the present invention, an endoscope inserting-operation program for inserting an inserting portion of an endoscope in a sample, comprises: a detecting step for detecting inserting-operation information of the endoscope inserting portion that is inserted in the sample; a storing step for storing standard inserting-operation information that is detected by the detecting unit and an endoscope image obtained by picking up an image of the sample at the position of a distal-end portion of the endoscope inserting portion upon the inserting operation with a correlation between; and a comparing and monitoring step for comparing the standard inserting-operation information stored in the storing step with the inserting-operation information obtained by the detecting step during the operation and of monitoring the situation of the inserting operation of the endoscope inserting portion.

Brief Description of the Drawings

Fig. 1 is a block diagram schematically showing the structure of a main structure portion provided to an endoscope system and an endoscope apparatus which are common to embodiments of the present invention;

Fig. 2 is a block diagram schematically showing an endoscope system and an endoscope apparatus according to the first embodiment;

Fig. 3 is a diagram showing a specific structure example of the endoscope system and the endoscope apparatus shown in Fig. 2;

Fig. 4A is a diagram showing the structure of an inserting-length measuring portion of an endoscope inserting portion;

Fig. 4B is a diagram showing the structure of an inserting-length measuring portion of an endoscope inserting portion according to a modification of Fig. 4A;

Fig. 5A is a diagram showing the structure of a turn angle measuring portion of the endoscope inserting portion;

Fig. 5B is a diagram showing the structure of a turn angle measuring portion of an endoscope inserting portion according to a modification of the structure shown in Fig. 5A;

Fig. 6A is a first explanatory diagram showing standard inserting-operation information stored in a storing unit shown in Fig. 1, and is a graph showing an angle of a bending portion to the inserting length of the endoscope inserting portion;

Fig. 6B is a second explanatory diagram showing the standard inserting-operation information stored in the storing unit shown in Fig. 1, and a graph showing a turn angle of the inserting portion to the inserting length of the endoscope inserting portion;

Fig. 6C is a third explanatory diagram showing the standard inserting-operation information stored in the storing unit shown in Fig. 1, and a graph showing the execution of an operating comment sentence, the display of a comment image, generation of comment voice (sound) in accordance with the inserting length of the endoscope inserting portion;

Fig. 7A is an explanatory diagram showing an operating example of a notifying unit according to the first embodiment, showing a first screen display example which is displayed on a display device shown in Fig. 3;

Fig. 7B is an explanatory diagram showing the operating example of the notifying unit according to the first embodiment, showing a second screen display example which is displayed on the display device shown in Fig. 3;

Fig. 7C is an explanatory diagram showing the operating example of the notifying unit according to the first embodiment, showing a third screen display example which is displayed on a display device shown in Fig. 3;

Fig. 7D is an explanatory diagram showing the operating example of the notifying unit according to the first embodiment, showing an angle of the operation instruction to the current inserting length of the inserting portion;

Fig. 8 is a flowchart showing an inserting-operation program for the endoscope system and the endoscope apparatus according to the first embodiment;

Fig. 9 is a block diagram schematically showing the structure of an endoscope system and an endoscope apparatus according to the second embodiment;

Fig. 10 is a structure diagram showing a structure example of an automatic inserting-operation unit according to the second embodiment;

Fig. 11A is a first explanatory diagram showing standard inserting-operation information stored in a storing unit shown in Fig. 9, and is a graph showing an angle of a bending portion to the time;

Fig. 11B is a second explanatory diagram showing the standard inserting-operation information stored in the storing unit shown in Fig. 9, and is a graph showing the inserting length of the endoscope inserting portion to the time;

Fig. 11C is a first explanatory diagram showing the standard inserting-operation information stored in the storing unit shown in Fig. 9;

Fig. 12 is a block diagram showing a specific structure example of the automatic inserting-operation unit shown in Fig. 10;

Fig. 13 is a block diagram showing the schematic structure of an endoscope system and an endoscope apparatus according to the third embodiment;

Fig. 14 is a diagram showing the structure of an endoscope system and an endoscope apparatus according to the third embodiment;

Fig. 15 is a diagram showing a screen display example of a virtual image display device shown in Fig. 14;

Fig. 16 is a diagram showing the structure of a medical treatment system according to the fourth embodiment;

Fig. 17 is a diagram showing the entire structure of an endoscope system and an endoscope apparatus according to the fifth embodiment;

Fig. 18 is an enlarged view showing a distal-end portion of the endoscope inserting portion shown in Fig. 17 and a probe distal-end side which is projected from the distal-end portion;

Fig. 19 is a diagram showing a display example of a monitor which displays an observed image obtained by an endoscope shown in Fig. 17;

Fig. 20 is a flowchart showing an image processing program in a navigation unit shown in Fig. 17;

Fig. 21 is an explanatory diagram showing a treatment tool which has a balloon on the distal-end side;

Fig. 22A is a diagram showing a monitor display example which displays, on a monitor, the observed image before swelling the balloon showing in Fig. 21;

Fig. 22B is a diagram showing a monitor display example of the observed image after swelling the balloon from the state shown in Fig. 22A;

Fig. 23 is a diagram showing the structure of, on the distal-end side, a probe which is used for an endoscope apparatus according to the sixth embodiment;

Fig. 24 is a cross-sectional view of a B-B line shown in Fig. 23;

Fig. 25A is a schematic diagram showing a relationship between three electrodes and the liquid surface of a conductive liquid when the gravity direction is on the lower right;

Fig. 25B is a schematic diagram showing a relationship between the three electrodes and the liquid surface of the conductive liquid when the gravity direction is on the right;

Fig. 26 is a diagram showing the structure, on the distal-end side, a probe according to a modification;

Fig. 27 is an explanatory diagram showing a state in which the probe shown in Fig. 26 is inserted from an inserting port of a treatment tool of the endoscope;

Fig. 28 is an enlarged perspective view showing an A portion or probe shown in Fig. 27;

Fig. 29 is an explanatory diagram showing, on the distal-end side, an inserting portion of the endoscope used for an endoscope apparatus according to the seventh embodiment;

Fig. 30 is a diagram showing a monitor display example in which a monitor displays an observed image obtained by an endoscope in the state shown in Fig. 29;

Fig. 31 is an explanatory diagram showing, on the distal-end side, the endoscope inserting portion when a rod member is pulled out to the distal-end portion side of the inserting portion and a fluid sealing portion is contact with the distal-end portion of the inserting portion;

Fig. 32 is a diagram showing a monitor display example of an observed image obtained by the endoscope in the state shown in Fig. 31;

Fig. 33 is an explanatory diagram showing, on the distal-end side of an inserting portion of the endoscope according to a modification;

Fig. 34 is a diagram showing a monitor display example of an observed image obtained by the endoscope shown in Fig. 33;

Fig. 35 is an explanatory diagram showing a state in which a balloon is projected in the upper oblique direction from a distal-end portion of an inserting portion of the endoscope in the upper oblique direction;

Fig. 36 is a diagram of a monitor display example showing an observed image obtained by the endoscope shown in Fig. 35;

Fig. 37 is an explanatory diagram showing, on the distal-end side, an inserting portion of an endoscope used for an endoscope apparatus according to the eighth embodiment;

Fig. 38 is an explanatory diagram showing, on the distal-end side, the endoscope inserting portion before swelling a balloon shown in Fig. 37;

Fig. 39 is an explanatory diagram showing a probe shown in Fig. 37 according to a modification;

Fig. 40 is an explanatory diagram showing a balloon swollen state;

Fig. 41 is a diagram showing a monitor display example of an observed image obtained by the endoscope shown in Fig. 37;

Fig. 42 is a graph showing the position and a range (size) of a spherical member shown on an image;

Fig. 43 is a model diagram showing a state in which a balloon is projected from the distal-end portion of the endoscope inserting portion;

Fig. 44 is an explanatory diagram of the model shown in Fig. 43;

Fig. 45 is a graph showing an observed image which is projected on the $z = f$ plane shown in Fig. 44;

Fig. 46 is an explanatory diagram showing a gravity sensor used for an endoscope apparatus according to the ninth embodiment;

Fig. 47 is a circuit block diagram including the gravity sensor shown in Fig. 46;

Fig. 48A is a front view schematically showing a state in which a conventional bronchoscope is inserted in the bronchus;

Fig. 48B is a side view schematically showing Fig. 48A;

Fig. 49A is a diagram showing a first image display example showing an endoscope image obtained by the conventional bronchoscope; and

Fig. 49B is a diagram showing a second image display example showing the endoscope image obtained by the conventional bronchoscope.

Best Mode for Carrying Out the Invention

Hereinbelow, a description is given of embodiments of the present invention with reference to the drawings.

(First embodiment)

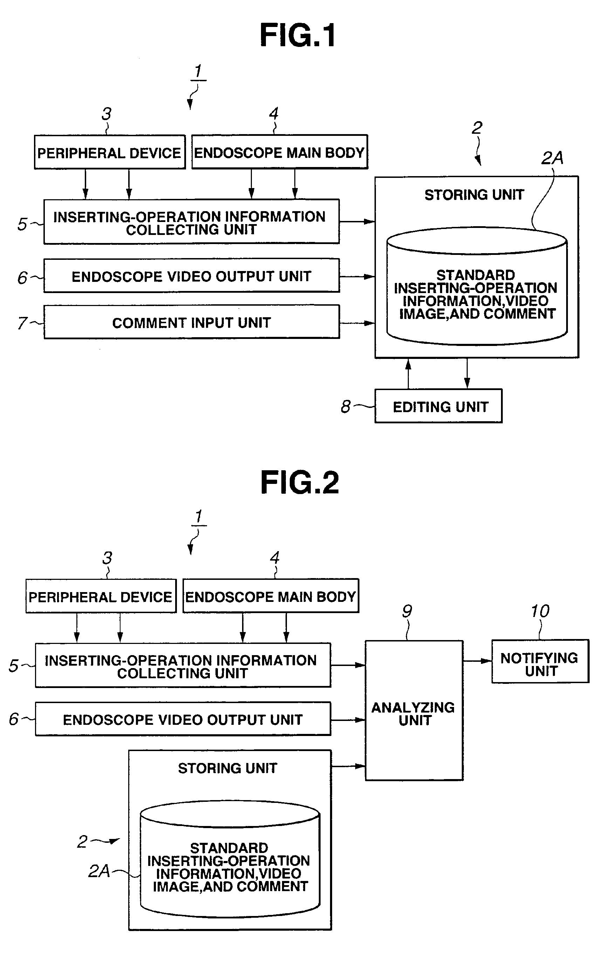

Figs. 1 to 8 show an endoscope system and an endoscope apparatus according to the first embodiment of the present invention.

Referring to Fig. 1, an endoscope system or an endoscope apparatus 1 comprises: a processing device 2 having a storing unit 2A; a peripheral device 3 necessary for endoscope diagnosis; an endoscope main body 4 having an endoscope inserting portion as a bronchoscope, which will be described later; an inserting-operation information collecting unit 5; an endoscope video output unit 6; a comment input unit 7; and an editing unit 8.

The inserting-operation information collecting unit 5 detects and collects, as standard inserting-operation information, information of inserting action by a skilled operator who inserts the endoscope inserting portion in the bronchi. The inserting-operation information collecting unit 5 comprises a detecting unit such as a sensor which can obtain the inserting-operation information via the peripheral device 3 and the endoscope main body 4.

The inserting-operation information includes an angle of the bending portion, a turn angle of the inserting portion, the inserting length of the inserting portion, the inserting speed of the inserting portion, a fixing state of the distal end of the inserting portion, a fixing state of a holding portion of the inserting portion, and the like. The inserting-operation information is detected by the detecting unit in the inserting-operation information collecting unit 5, and is captured. The inserting-operation information collecting unit 5 supplies the captured inserting-operation information to the processing device 2. The processing device 2 detects a relative positional relationship between the distal-end portion of the inserting portion and the lumen in the body cavity as a sample based on the information including the angle of the bending portion, the turn angle of the inserting portion, and the inserting length of the inserting portion, and correlates the endoscope image at the position with the corresponding position (particularly, based on the inserting length of the inserting portion).

The endoscope video output unit 6 outputs, to the processing device 2, the endoscope image (live image) which is obtained from a distal-end portion of an endoscope inserting portion 4A in a bronchoscope 14.

The comment input unit 7 comprises a character input unit, a voice input unit, and an image input unit (which are not shown). The comment input unit 7 creates comment information via each input unit and outputs the created information to the processing device 2 when the inserting-operation information has characteristic operation and a note.

The processing device 2 has the storing unit 2A having a large memory capacity, and stores, in the storing unit 2A, the inserting-operation information obtained by the inserting-operation information collecting unit 5 and the endoscope image from the endoscope video image output unit 6, as time-series data. In this case, the processing device 2 stores the inserting-operation information and the endoscope image every time with a correlation therebetween. Here, the processing device 2 detects the relative positional relationship between the sample and the distal-end portion of the endoscope inserting-portion based on the inserting-operation information, and the storing unit 2A stores positional relationship information and the endoscope image with the correlation therebetween. Namely, the processing device 2 correlates the relative positional information on the distal-end portion of the endoscope inserting-portion and the sample, the endoscope image at the position, and the inserting-operation information at the position with each other and stores the correlated information in the storing unit 2A.

Further, when the comment input unit 7 supplies the comment information such as the inserting-operation information, the processing device 2 can add and restore the comment information to the portion corresponding the time-series data which constitutes the inserting-operation information and the endoscope image correlated therebetween and which is stored in the storing unit 2A.

The editing unit 8 is connected to the processing device 2. The editing unit 8 reads stored information which is stored in the storing unit 2A of the processing device 2, erases and rearranges unnecessary information, and re-records the information.

Next, a description is given of the structure of the endoscope system or the endoscope apparatus 1 according to the first embodiment with reference to Fig. 2.

In addition to the structure shown in Fig. 1, the endoscope system or the endoscope apparatus 1 has an analyzing unit 9 and a notifying unit 10 as a monitoring control unit shown in Fig. 2.

The analyzing unit 9 as the monitoring control unit is connected to the inserting-operation information collecting unit 5, the endoscope video output unit 6, and the processing device 2, and receives the live inserting-operation information and endoscope image, the standard inserting-operation information as the stored information, and the endoscope image with the correlation with the inserting-operation information (the endoscope image which is obtained by picking up the sample at the position of the distal-end portion of the inserting-portion detected based on the inserting-operation information). The operator operates the bronchoscope and, thus, the analyzing unit 9 collects in real time the inserting-operation information and the endoscope image from the inserting-operation information collecting unit 5 and the endoscope video output unit 6. Further, the analyzing unit 9 sequentially analyzes the collected inserting-operation information and endoscope image by comparing with the standard inserting-operation information and the endoscope image correlated with the inserting-operation information, which are stored in the storing unit 2A in the processing device 2. The comparison result is outputted to the notifying unit 10 so as to monitor the inserting operation on the endoscope inserting portion performed by the operator.

The notifying unit 10 comprises a display unit and a voice playing unit. The notifying unit 10 presents, to the operator, the standard inserting-operation information as the procedure for the inserting operation by displaying or playing the comparison result from the analyzing unit 9 by using the character, video image, and voice.

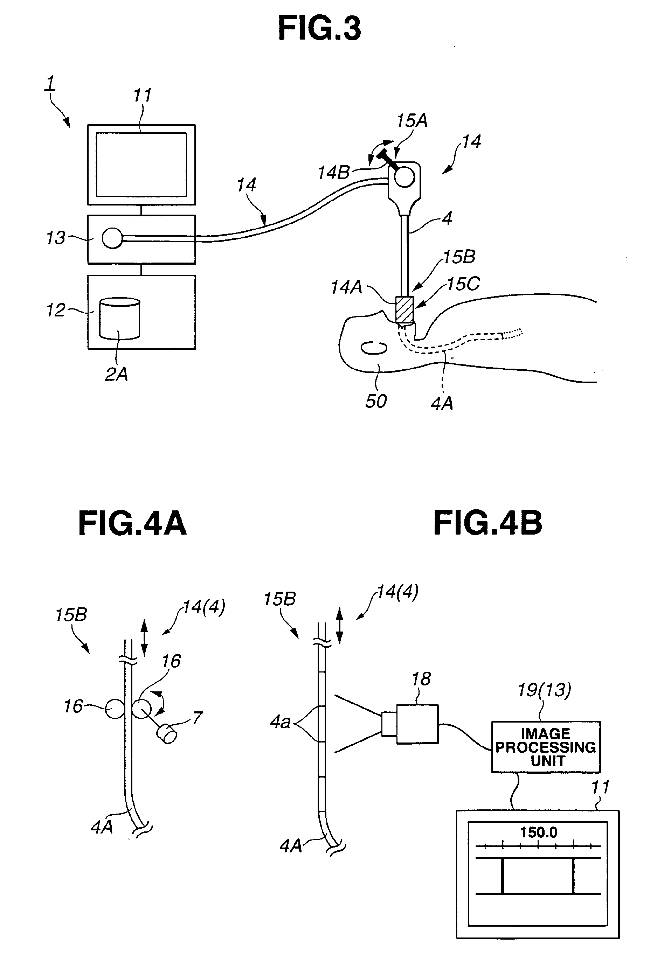

Referring to Fig. 3, the endoscope system or the endoscope apparatus 1 comprises: a display device 11 serving as the notifying unit 10 having a speaker and a monitor; an inserting-operation amount processing device 12 having the processing device 2 including the storing unit 2A and the analyzing unit 9; a bronchoscope 14 having an inserting portion 4A; a mouse piece 14A which holds the endoscope inserting portion 4A so as to be able to insert safely and smoothly the endoscope inserting portion 4A to the body cavity such as the bronchi via the mouse of a patient 50; a video processor 13 for endoscope including the endoscope video output unit 6 which processes endoscope image information from the bronchoscope 14; an angle measuring unit 15A of a bending portion which measures an angle of he bending portion and which is arranged to the bronchoscope 14 as the inserting-operation information collecting unit 5; an inserting-length measuring unit 15B which measures the inserted length of the inserting portion (which can detect the inserting speed); and a turn angle measuring unit 15C which measures the turn angle of the inserting portion.

In the case of the diagnosis, in the bronchoscope 14, the mouth piece 14A held by the mouth of the patient holds the insertion of the endoscope inserting portion 4A.

An operating lever 14B for adjusting the angle of the bending portion of the endoscope inserting portion 4A is arranged near the operating portion of the bronchoscope 14. Upon adjusting the angle of the bending portion by using the operating lever 14B, the angle measuring portion 15A arranged near the operating lever 14B measures the angle which is formed by bending the bending portion by the operator.

The endoscope video processor 13 processes the endoscope image data from the bronchoscope 14 by the endoscope video output unit 6, and outputs the processed data to the inserting-operation amount processing device 12. Further, the endoscope video processor 13 captures the measured results from the angle measuring unit 15A of the bending portion, the inserting-length measuring unit 15B of the inserting portion (measuring the inserting speed of the inserting portion if necessary), and the turn angle measuring unit 15C of the inserting portion, and outputs the captured results to the inserting-operation amount processing device 12 similarly.

The inserting-operation amount processing device 12 executes the processing of the above-mentioned processing device 3 and the analyzing unit 9 as the monitoring control unit. The inserting-operation amount processing device 12 obtains the inserting-operation information on the inserting portion performed by the operator, sequentially analyzes the obtained inserting-operation information and endoscope image by comparing with the standard inserting-operation information and the endoscope image (storing information) correlated with the inserting-operation information, which are stored in the string unit 2A. Further, the inserting-operation amount processing device 12 monitors the operating situation, outputs the comparison result to the display device 11, and displays the output.

The display device 11 has a monitor and a speaker. Under the control of the inserting-operation amount processing device 12, the display device 11 notifies the operator of the inserting-operation situation of the bronchoscope 14 and the operating instruction by the character, video image, and voice based on the analyzing and comparison results. Thus, the standard inserting-operation information and the procedure are reflected to the endoscope operation. A description will be given of an example of instructing the notification by the display device 11 as the notifying unit 10 later.

Next, a description is given of an example of the specific structure of the inserting-length measuring unit 15B of the inserting portion and the turn angle measuring unit 15C of the inserting portion with reference to Figs. 4A to 5B.

First, a description is given of an example of the structure of the inserting-length measuring unit 15B of the inserting portion.

Referring to Fig. 4A, the inserting-length measuring unit 15B of the inserting portion is arranged to be contact with the peripheral surface of the endoscope inserting portion 4A which is inserted in the bronchus. The inserting-length measuring unit 15B of the inserting portion comprises a pair of rollers 16 which can freely be rotated in the moving direction of the endoscope inserting portion 4A, and a potentiometer 17 as a measuring unit which measures the amount of rotation of the rollers 16. The measuring unit for measuring the amount of rotation of the rollers 16 is not limited to the potentiometer 17 and may be another angle measuring device which can measure the rotating angle of the rollers 16. In this case, the inserting speed of the inserting portion can be measured by measuring the inserting-length per unit time.

In the inserting-length measuring unit 15B with the above structure, the pair of rollers 16 is rotated in accordance with the pull-in operation and the pull-out operation of the endoscope inserting portion 4A. Further, interlocking with the rotation, the potentiometer 17 rotates, thus, the amount of rotation of the potentiometer is measured based on the amount of rotation of the rollers 16, the measured result is converted into an electrical signal, and the converted signal is outputted to the inserting-operation amount processing device 12. On the other hand, the inserting-operation amount processing device 12 obtains the inserting length of the inserting portion based on the measured result. In this case, the inserting length of the inserting portion is obtained by the following (formula 1).

The inserting length of the inserting portion

= k (predetermined conversion coefficient) × the amount

of potentiometer rotation                                                    (Formula 1)

Next, another modification is shown.

Referring to Fig. 4B, the inserting-length measuring unit 15B of the inserting portion comprises: one or a plurality of video cameras 18 which pick up in real time an image of the movement of markers 4a arranged at equal intervals on the peripheral surface of the endoscope inserting portion 4A; and an image processing unit 19 which performs in real time the image processing of the image pick-up signal from the video camera 18 and calculates the amount of movement of the markers 4a on the screen (on the display screen of the display device 11). The image pick-up unit for picking up the movement of the markers 4a in real time is not limited to the video camera 18 and may be a two-dimensional image pick-up device such as a CIS.

The inserting-length measuring unit 15B of the inserting portion photographs in real time the makers 4a which move by the video camera 18 in accordance with the pull-in operation and the pull-out operation of the endoscope inserting portion 4A. The image processing unit 19 performs in real time the image processing of the image pick-up signal from the video camera 18, calculates the amount of movement of the markers 4a on the screen (on the display screen of the display device 11), and obtains the inserting length of the inserting portion based on the calculated result.

Next, a description is given of an example of the structure of the turn angle measuring unit 15C of the inserting portion.

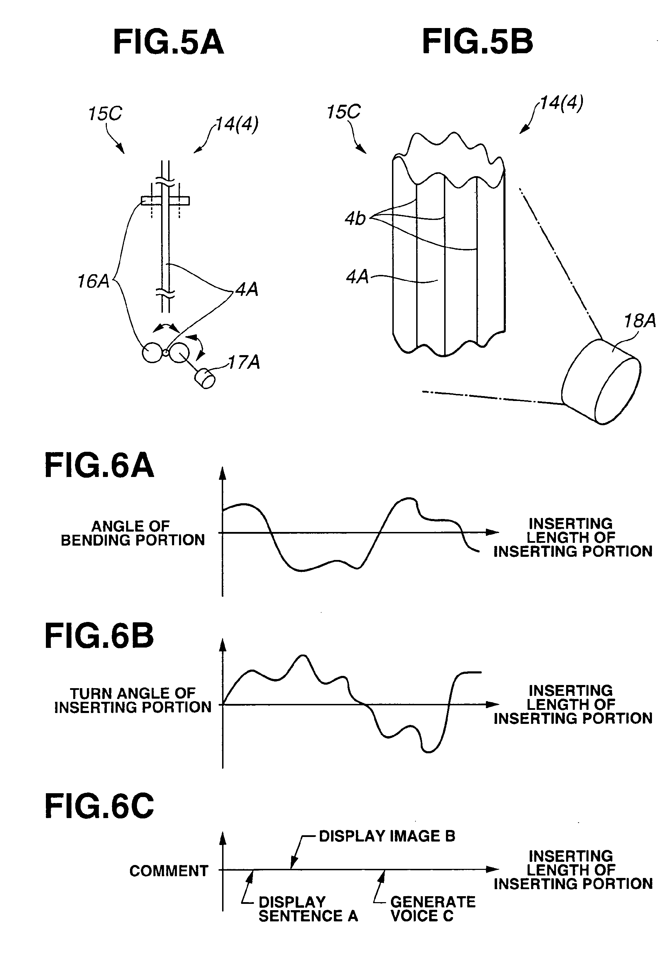

Referring to Fig. 5A, the turn angle measuring unit 15C of the inserting portion is arranged to be contact with the peripheral surface of the endoscope inserting portion 4A which is inserted in the bronchi. Further, the turn angle measuring unit 15C of the inserting portion comprises a pair of rollers 16A which can freely be rotated in the rotating direction of the endoscope inserting portion 4A, and a potentiometer 17A as a measuring unit which measures the amount of rotation of the rollers 16A. The measuring unit for measuring the amount of rotation of the rollers 16A is not limited to the potentiometer 17A and may be another angle measuring device which can measure the rotating angle of the rollers 16A.

In the turn angle measuring unit 15C of the inserting portion, the endoscope inserting portion 4A is turned in accordance with the pull-in operation or pull-out operation of the endoscope inserting portion 4A, the pair of rollers 16A are rotated, and the potentiometer 17A is rotated interlocking with the rotation. Consequently, the turn angle measuring unit 15C of the inserting portion measures the amount of potentiometer rotation based on the amount of roller rotation, converts the measured result into an electrical signal, and outputs the converted signal to the inserting-operation amount processing device 12. The inserting-operation amount processing device 12 obtains the turn angle of the inserting portion based on the measured result. In this case, the turn angle of the inserting portion is obtained by the following (Formula 2).

The turn angle of the inserting portion

= h (predetermined converting coefficient) × the amount

of potentiometer rotation                                                    (Formula 2)

Next, another modification will be shown. Referring to Fig. 5B, similar to the inserting-length measuring unit 15B of the inserting portion, this modification comprises: one or a plurality of video cameras 18A which pick up in real time images of the movement of markers 4b arranged at equal intervals in the same direction as the inserting direction of the endoscope inserting portion 4A on the peripheral surface of the endoscope inserting portion 4A; and the image processing unit (although not shown, having the similar structure with that shown in Fig. 4B) 19 which performs in real time the image processing of the image pick-up signal from the video camera 18A and calculates the amount of movement of the markers 4b on the screen (on the display screen of the display device 11). According to the modification, the image pick-up unit for picking up the images of the movement of the markers 4b in real time is not limited to the video camera 18A and may be a two-dimensional image pick-up unit such as a CIS.

In the turn angle measuring unit 15C of the inserting portion with the above-mentioned structure, the endoscope inserting portion 4A is turned in accordance with the pull-in operation or pull-out operation of the endoscope inserting portion 4A, then, the video camera 18A picks up the images of the amount of movement of the markers 4b, the image pick-up signal from the video camera 18A is subjected to the image processing in real time, and the amount of movement of the markers 4b on the screen (screen of the display device 11) is calculated, and the turn angle of the inserting portion is obtained based on the calculated result.

Next, a description is given of the operation of the endoscope system or endoscope apparatus 1 according to the first embodiment with reference to Figs. 6A to 8.

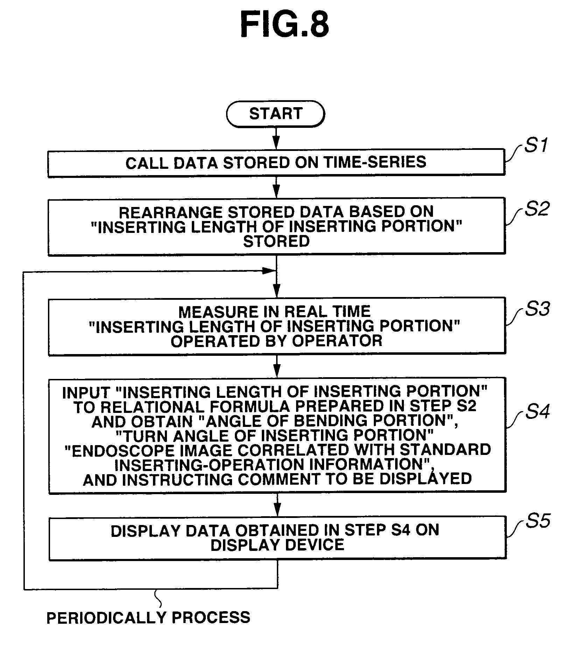

It is assumed that the operator performs the diagnosis of the bronchi by using the endoscope system or endoscope apparatus 1 according to the first embodiment. A control unit (not shown) of the inserting-operation amount processing device 12 starts the processing routine of an endoscope inserting-operating program shown in Fig. 8. That is, in the processing in step S1, stored information in the storing unit 2A in the inserting-operation amount processing device 12 (standard inserting-operation information and the endoscope image at the distal-end position of the inserting portion upon the operation) is read out, and the processing routine shifts to step S2.

In the processing in step S2, the control unit rearranges the stored data based on the inserting length of the inserting portion. For example, examples of the stored data obtained by the above processing are shown in Figs. 6A and 6B.

Referring to Fig. 6A, the inserting-operation information indicates the angle of the bending portion shown on the ordinate in accordance with the inserting length of the inserting portion shown on the abscissa. Referring to Fig. 6B, the inserting-operation information indicates the turn angle of the inserting portion on the ordinate with respect to the inserting length of the inserting portion shown on the abscissa. Similarly, the endoscope image stored being correlated with the standard inserting-operation information and the comment information are rearranged based on the inserting length of the inserting portion if necessary. For example, referring to Fig. 6C, data for instructing the inserting operation is formed to execute the display operation of a sentence A and an image B, the generation (sound) of voice C and the like, in accordance with the inserted length of the inserting length on the abscissa.

In this case, the data for instructing the inserting operation is calculated by a function of the inserting length of the inserting portion as shown by the following Formula 3 and Formula 4.

$$\text{The angle of the bending portion} = f \text{ (inserting length of the inserting portion)} \quad \text{(Formula 3)}$$

$$\text{The turn angle of the inserting portion} = g \text{ (inserting length of the inserting portion)} \quad \text{(Formula 4)}$$

The endoscope image and instructing comment information (including sentence, voice, and image) are calculated by a similar function of the inserting length of the inserting portion.

The control unit shifts the processing to that in step S3. In the processing in step S3, the inserting length of the inserting portion which is currently operated by the operator is measured in real time by using the inserting-length measuring unit 15B of the inserting portion, and shifts the processing to that in step S4.

In the processing in step S4, the control unit uses the relational formulae (Formula 3 and Formula 4) provided in the processing in step S2, inputs the inserting length of the inserting portion measured in step S3, obtains the angle of the bending portion, turn angle of the inserting portion, endoscope image correlated with the inserting-operation information, and the instructing comment information, and then shifts the processing to that in step S5.

In the processing in step S5, the control unit outputs and displays the data obtained in step S4 to the display device 11 (refer to Fig. 3) as the notifying unit 10.

After that, the processing routine returns to step S3 whereupon the processing routine in step S3 or step S5 is periodically executed, and an instruction for the inserting operation is supplied to the operator.

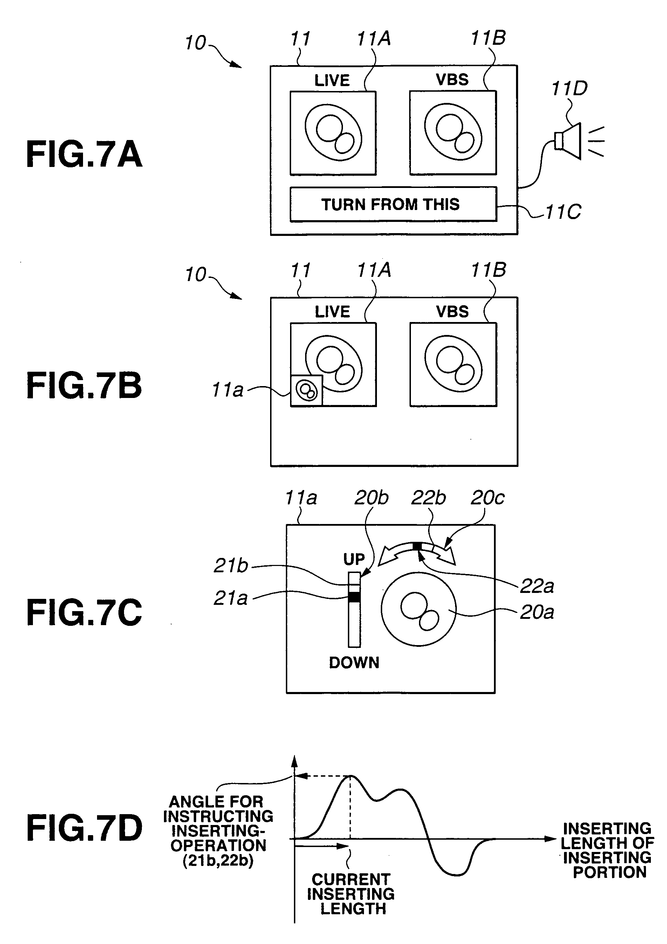

Figs. 7A to 7C show display examples presented to the display device 11 as a result of the processing in step S5.

Referring to Fig. 7A, the control unit in the inserting-operation amount processing device 12 of the inserting portion displays at least three multi-screens on the screen of the display device 11, thereby notifying the operator of the instruction for the inserting operation. That is, two screens 11A and 11B are displayed as a multi-screen on the top of the screen of the display device 11, one screen 11C is displayed as a multi-screen on the bottom of the screen, the endoscope image (live image) obtained by the endoscope inserting portion 4A of the bronchoscope is displayed on the screen 11A, and the virtual endoscope image (VBS image) in the bronchus is displayed based on CT image data. Simultaneously, the instruction for the inserting operation is displayed as guidance by the characters on a screen 11C on the bottom of the screen, and the characters are reproduced as voice via a speaker 11D. That is, the characters and voice instruct the angle of the bending portion or the turn angle of the inserting portion by viewing the live image and the VBS image to the operator based on the inserting length of the inserting portion.

When the instruction comment information is present or the comment information is added to the editing unit 8 shown in Fig. 1, referring to Fig. 6C, the comment information (including the sentence, voice, and image) is displayed on the corresponding screen on the screen of the display device 11 or is reproduced as voice via the speaker 11D, based on the inserting length of the inserting portion as the standard.

In the example shown in Fig. 7A, instead of the virtual endoscope image displayed on the screen 11B, the endoscope image is displayed based on the inserting length of the inserting portion stored having the correlation with the standard inserting-operation information.

Further, in the example shown in Fig. 7A, the screen 11B shown in Fig. 7C may display simultaneously, to present to the operator, a bending-portion angle display unit 20b which displays a display bar 21a indicating the current angle of the bending portion and a display bar 21b indicating the operating instructing angle as the stored standard inserting-operation information; a turn angle display unit 20c of the inserting portion which displays a display bar 22a indicating the current angle of the inserting portion and a display bar 22b indicating the stored standard inserting-operation instructing angle; and a display unit 20a which displays the endoscope image at the position of the distal-end portion of the inserting-portion in the inserting operation with the correlation with the standard inserting-operation information.

According to the first embodiment, referring to Fig. 7B, on the screen 11A which displays the live image, a slave screen 11a may display the endoscope image based on the same inserting length stored being correlated with the standard inserting-operation information. Thus, the instruction for the inserting operation can be presented in more detail. Therefore, the endoscope apparatus 1A can insert the inserting portion of the bronchoscope 14 without fail.

Further, referring to Fig. 7C, as another display example of the instruction for the inserting operation, the slave screen 11a may simultaneously display, to present to the operator, a bending-operation angle display unit 20b which displays a display bar 21a indicating the current angle of the bending portion and a display bar 21b indicating the angle for instructing the operation as the stored standard inserting-operation information, a turn angle display unit 20c of the inserting portion which displays a display bar 22a indicating the current turn angle of the inserting portion and a display bar 22b indicating the standard stored angle for instructing the inserting operation, and a display unit 20a which displays the endoscope image at the position of the distal-end portion of the inserting portion in the inserting operation correlated with the standard inserting-operation information. In this case, referring to Fig. 7D, the angle for instructing the inserting operation is displayed with respect to the current inserting length of the inserting portion. When the current angle is excessively different from the angle for instructing the inserting operation, the control operation may be performed such that the display device 11 displays or reproduces the character or voice indicating an alarm message.

As mentioned above, according to the first embodiment, the instruction for the inserting operation can be presented based on the standard inserting-operation information of the inserting portion. Thus, the endoscope can be inserted to the target position with precision for a short image.

(Second embodiment)

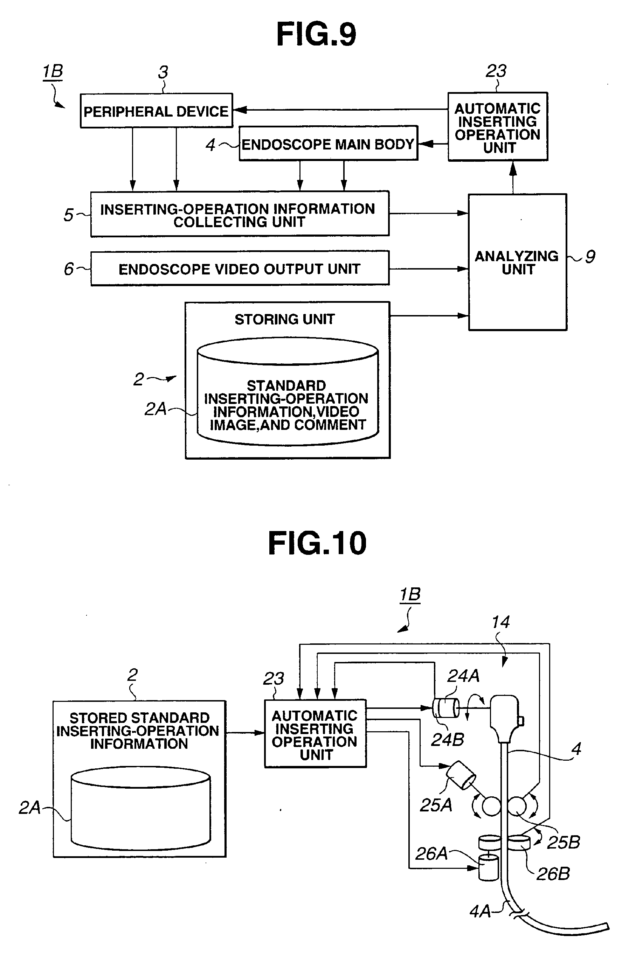

Figs. 9 to 12 show an endoscope system or an endoscope apparatus according to the second embodiment of the present invention. Referring to Figs. 9 to 12, the same components as those of the endoscope apparatus 1 according to the first embodiment are designated by the same reference numerals, and only different portions are described.

The endoscope system or endoscope apparatus according to the second embodiment is structured by adding an automatic inserting-operation unit 23 which automatically controls the inserting operation based on the instruction for the inserting operation using the notifying unit 10 according to the first embodiment. Other structures are the same as those of the endoscope apparatus 1 according to the first embodiment.

Referring to Fig. 9, in an endoscope apparatus (system) 1B according to the second embodiment, the automatic inserting-operation unit 23 is arranged among the analyzing unit 9, the peripheral device 3, and the bronchoscope 14. The automatic inserting-operation unit 23 performs the same processing contents as those of the notifying unit 10 according to the first embodiment, and automatically controls various operations of the bronchoscope 14 and another peripheral device 3 based on the analysis result from the analyzing unit 9.

That is, the automatic inserting-operation unit 23 automatically controls, based on the analysis result, the inserting operation of the inserting portion of the bronchoscope 14. Similarly to the first embodiment, the inserting operation of the inserting portion of the bronchoscope 14 includes the angle operation of the bending portion, the turn operation of the inserting portion, the inserting operation of the inserting portion, the operation for fixing and resetting the distal end of the inserting portion, and the operation for fixing and resetting a holding portion of the inserting portion.

In this case, similarly with the first embodiment, the display device 11 may display the situation of the inserting operation of the bronchoscope 14 which is automatically inserted.

Next, the structure for the automatic inserting operation of the bronchoscope 14 is shown in Fig. 10. An automatic inserting device has a plurality of driving units which are controlled by the automatic inserting-operation unit 23 and which perform the inserting operation of the inserting portion of the bronchoscope 14.

The plurality of driving units comprise various motors including an angle adjusting motor 24A of the bending portion, an inserting-length adjusting motor 25A of the inserting portion, and a turn angle adjusting motor 26A of the inserting portion.

The angle adjusting motor 24A is constitutionally integrated with the angle measuring unit 24B of the bending portion in the bronchoscope 14, and performs the angle operation of the bending portion in the endoscope inserting portion 4A by transmitting rotating force by connecting its rotating shaft to an angle adjusting mechanism (not shown) of the bending portion in the bronchoscope 14. The angle measuring unit 24B of the bending portion always detects the angle of the bending portion, and outputs the detected result to the automatic inserting-operation unit 23.

The inserting-length adjusting motor 25A of the inserting portion is arranged to be contact with the peripheral surface of the endoscope inserting portion 4 which is inserted in the bronchi, and is directly connected to a pair of rollers which rotate in the moving direction of the endoscope inserting portion 4A so as to apply driving force to the rollers. The inserting-length measuring unit 25B of the inserting portion is arranged near the inserting-length adjusting motor 25A of the inserting portion and measures the amount of rotation of the inserting-length adjusting motor 25A of the inserting portion so as to always output the measured result to the automatic inserting operation unit 23.

The turn angle adjusting motor 26A of the inserting portion is arranged to be contact with the peripheral surface of the endoscope inserting portion 4A which is inserted in the bronchi. Further, the turn angle adjusting motor 26A of the inserting portion is directly connected to a pair of rollers that rotate in the rotating direction (turn direction) of the endoscope inserting portion 4A so as to apply driving force to the rollers. The turn angle measuring unit 26B of the inserting portion and is arranged near the turn angle adjusting motor 26A of the inserting portion and measures the amount of rotation of the turn angle adjusting motor 26A of the inserting portion so as to always output the measured result to the automatic inserting-operation unit 23.

The automatic inserting-operation unit 23 recognizes the current angle of the bending portion of the bronchoscope 14, the inserting length of the inserting portion, and the turn angle of the inserting portion based on the measured results from the angle measuring unit 24B of the bending portion, the inserting-length measuring unit 25B of the inserting portion, and the turn angle measuring unit 26B of the inserting portion, and controls the rotating driving of the angle adjusting motor 24A of the bending portion, the inserting-length adjusting motor 25A of the inserting portion, and the turn angle adjusting motor 26A of the inserting portion.

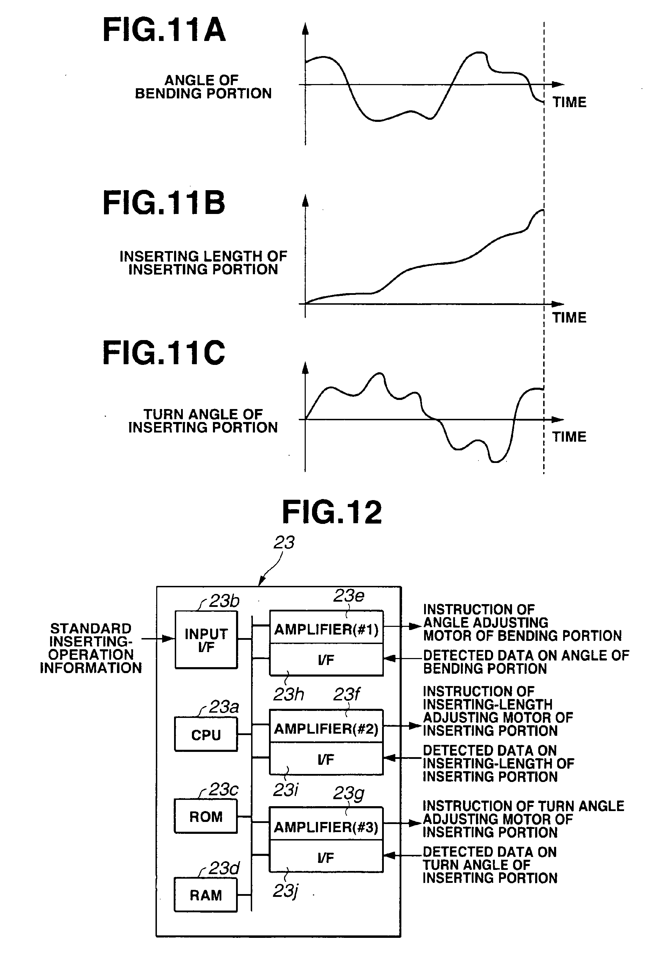

That is, according to the second embodiment, Figs. 11A to 11C show examples of the standard inserting-operation information read from the storing unit 2A. The automatic inserting-operation unit 23 controls the rotation driving of the angle adjusting motor 24A of the bending portion, the inserting-length motor 25A of the inserting portion, and the turn angle adjusting motor 26A of the inserting portion so as to substantially match the operating information shown in Figs. 11A to 11C.

Fig. 12 shows the structure of the automatic inserting-operation unit 23.

Referring to Fig. 12, the automatic inserting-operation unit 23 comprises: a CPU 23a as a control unit which controls the reading of the storing unit 2A and the various driving control for the bronchoscope 14; an input interface (hereinafter, referred to as an I/F) 23b which captures the standard inserting-operation information from the storing unit 2A; a ROM 23c which stores a program necessary for the automatic inserting operation and the operating information such as the captured inserting-operation information; a RAM 23d which temporarily stores, as a working area for comparison operation processing, the measured result of inserting the inserting portion of the bronchoscope 14 and the standard inserting-operation information; a first amplifier 23e which amplifies and outputs a driving signal for controlling the driving of the angle adjusting motor 24A of the bending portion; an I/F 23h which captures the measured result from the angle measuring unit 24B of the inserting portion; a second amplifier 23f which amplifies and outputs a driving signal for controlling the driving of the inserting-length adjusting motor 25A of the inserting portion; an I/F 23i which captures the measured result from the inserting-length measuring unit 25B of the inserting portion; a third amplifier 23g which amplifies and outputs a driving signal for controlling the deriving of the turn angle adjusting motor 26A of the inserting portion; and an I/F 23j which captures the measured result from the turn angle measuring unit 26B of the inserting portion.

According to the second embodiment, the endoscope apparatus (system) 1B recognizes the current angle of the bending portion, inserting length of the inserting portion, and turn angle of the inserting portion of the bronchoscope 14, based on the measured results from the angle measuring unit 24B of the bending portion, the inserting-length measuring unit 25B of the inserting portion, and the turn angle measuring unit 26B of the inserting portion, and simultaneously controls the rotation driving of the angle adjusting motor 24A of the bending portion, the inserting-length motor 25A of the inserting portion, and the turn angle adjusting motor 26A of the inserting portion such that the inserting state of the endoscope inserting portion 4A becomes the inserting-operation state based on the standard inserting-operation information read from the storing unit 2A. Thus, the endoscope apparatus (system) 1B according to the second embodiment automatically performs the inserting operation of the inserting portion of the bronchoscope 14 based on the standard inserting-operation information.

According to the second embodiment, the description is given of the case in which the automatic inserting-operating unit 23 automatically performs the inserting operation of the inserting portion in the bronchoscope 14. However, the operation may be switched between an automatic mode and a manual mode and the operating instruction may be presented to the operator by switching to the manual mode upon needing the manual operation, similarly to the according to the first embodiment.

Therefore, according to the second embodiment, the inserting portion of the bronchoscope 14 can automatically be inserted based on the standard inserting-operation information and, then, the endoscope can be inserted to the target portion with precision for a short time, irrespective of the operator's skill.

According to the second embodiment, the standard inserting-operation information is reflected to the inserting operation of the inserting portion of the bronchoscope 14 by the operator during the endoscope operation. Thus, the operator has an experience in which he/she uses the standard inserting-operation information by the endoscope apparatus for training. Consequently, the standard inserting-operation information can effectively be used. The above-mentioned structure will be described hereinbelow.

(Third embodiment)

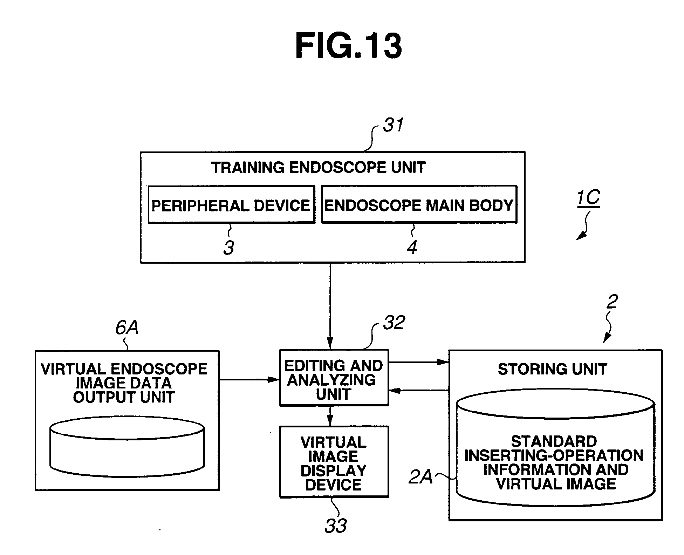

Figs. 13 to 15 show an endoscope system or the endoscope apparatus according to the third embodiment of the present invention. As shown in Figs. 13 to 15, the same components as those according to the first embodiment are designated by the same references, a description thereof is omitted, and only different portions are described.

According to the third embodiment, in place the bronchoscope 14 according to the first embodiment, an endoscope apparatus (system) 1C comprises a training endoscope unit 31 constituting a training bronchoscope having the similar structure with the first embodiment. Further, the endoscope apparatus (system) 1C comprises a virtual endoscope image data output unit 6A which outputs virtual endoscope image data, in place of the endoscope video output unit 6, and an editing and analyzing unit 32 which can perform the similar processing of the editing unit 8 and the analyzing unit 9. Other structures are the same as those according to the first embodiment.

Referring to Fig. 13, in the endoscope apparatus (system) 1C according to the third embodiment, the storing unit 2A of the processing device 2 stores the standard inserting-operation information and the virtual endoscope image data. Similarly to the first embodiment, the editing and analyzing unit 32 reads, rearranges, or edits the stored data.

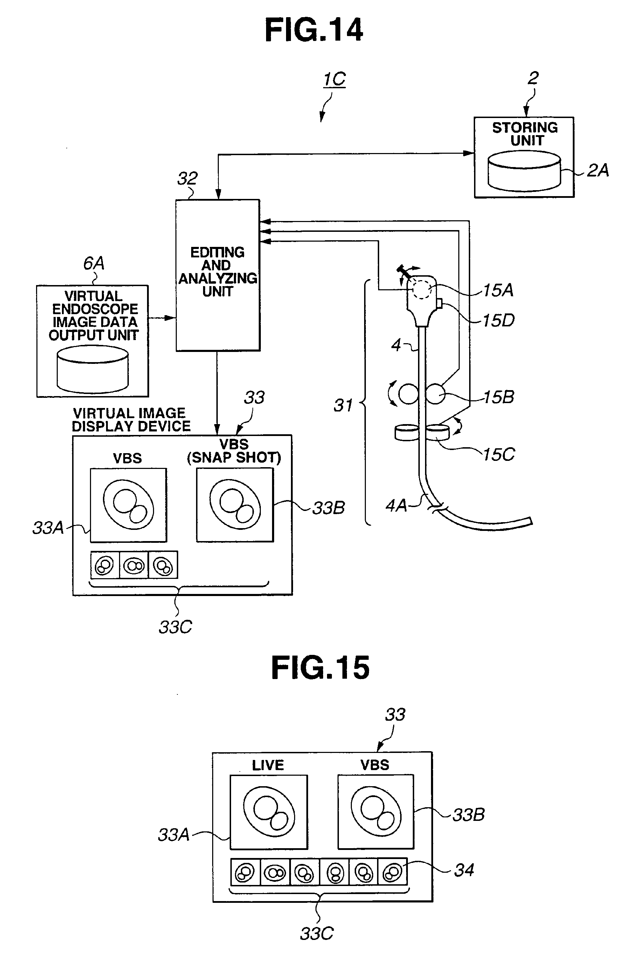

The training endoscope unit 31 has a bronchoscope having the peripheral device 3 and the endoscope inserting portion 4A and further has the same structure of that of the bronchoscope 14 according to the first embodiment. Referring to Fig. 14, in detail, the training endoscope portion 31 comprises an angle measuring unit 15A of the bending portion, an inserting-length measuring unit 15B of the inserting portion, and an angle measuring unit 15C of the inserting portion. The training endoscope unit 31 outputs the inserting-operation information obtained from the measuring units 15A, 15B, and 15C to the editing and analyzing unit 32. A freeze button 15D is arranged near a hand operating portion of the training endoscope. When the freeze button 15D is pressed, the virtual endoscope image displayed at that time is displayed as a snap shot.

The virtual endoscope image data output unit 6A generates the endoscope image (VBS image) in the bronchi based on CT image data, and outputs the generated image to the editing and analyzing unit 32.

The virtual image display device 33 has the same structure as that of the display device 11 used according to the first embodiment. The virtual image display device 33 displays at least three multi-screens, thus to display the virtual endoscope image as the result of the inserting operation of the training endoscope unit 31.

The editing and analyzing unit 32 obtains the inserting-operation information based on the result of measuring the inserting portion by the operator with the training endoscope unit 31, sequentially compares and analyses the obtained inserting-operation information and the virtual endoscope image with the storing image stored in the storing unit 2A (standard inserting-operation information and the endoscope image), monitors the inserting-operation situation, outputs the comparison result to the virtual image display device 33, and display the output.

For example, referring to Fig. 7A, in this case, in the editing and analyzing unit 32, the virtual image display device 33 displays two screens 33A and 33B as a multi-screen on the top of the screen thereof, and displays one screen 33C on the bottom of the screen as a multi-screen. The screen 33A displays the virtual endoscope image (VBS image) from the virtual endoscope image data output unit 6A based on the result of measuring the inserting operation of the training endoscope unit 31. The screen 33B displays the snap shot of the virtual endoscope image (VBS image) displayed upon pressing the freeze button 15D of the training endoscope unit 31. Simultaneously, the screen 33C on the bottom of the screen displays as a list a past snap shot (VBS image) 34 from the left to the right in the drawing in order of the shorter inserting-length of the inserting portion (refer to Fig. 15).

List display information of the snap shot on the screen 33C is stored in the storing unit 2A in the processing device 2 under the control of the editing and analyzing unit 32. Similarly to the first embodiment, the list display information is displayed on the virtual display device 33 as the notifying unit 10, thereby presenting the operating instruction to the operator who inserts the training endoscope unit 31.

Other structures and operations are the same as those according to the first embodiment.

Therefore, according to the third embodiment, the operator can have the experience of using the standard inserting-operation information with the training endoscope device. Thus, the standard inserting-operation information and the procedure can effectively be used. For example, the use of the training endoscope device as an education system excessively contributes to improving the inserting skill of the operator.

In the endoscope apparatus (system) 1C according to the third embodiment, similarly to the second embodiment, the editing and analyzing unit 32 automatically operates the training endoscope unit 31 based on the standard inserting-operation information. Thus, the operator who operates the training endoscope unit 31 may have the experience of the inserting method similar to the standard inserting-operation information and the procedure.

According to the first to third embodiments, the description is given of the case in which the instruction for the inserting-operation is supplied in accordance with the standard inserting-operation information while displaying the VBS image as well as the live image. However, the present invention is not limited to this and the instruction for the inserting operation may be presented while displaying only the live image.

An endoscope apparatus shown in the following can easily detect the gravity direction of the distal-end portion of the inserting portion. The endoscope with the above-mentioned structure can be applied to an endoscope system and an endoscope apparatus according to the first to third embodiments.

(Forth embodiment)

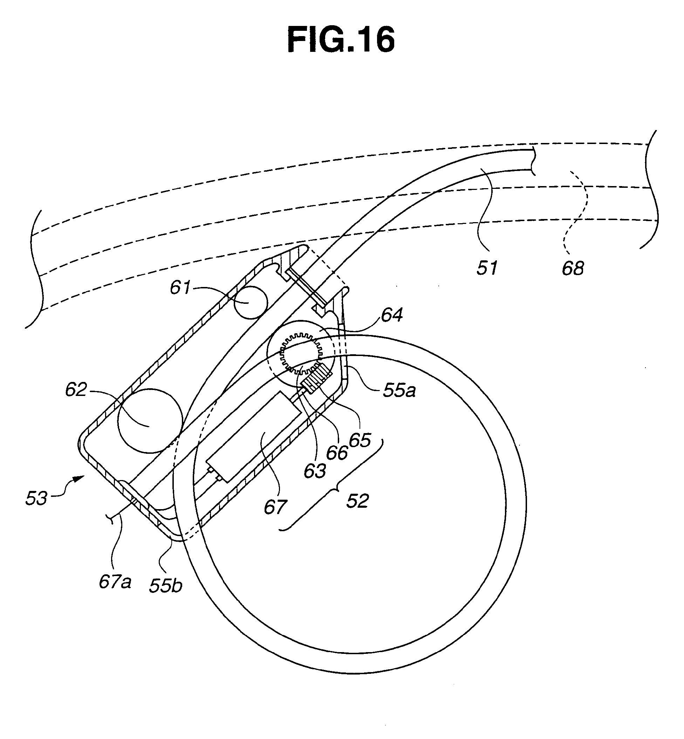

Fig. 16 shows the fourth embodiment of the present invention.

The endoscope inserting portion is used as the examples according to the first to third embodiments. However, as an applying example for inserting a catheter for blood vessel cure, a medical treatment tool having a long inserting portion is used according to the fourth embodiment.

Referring to Fig. 16, a medical treatment system according to the fourth embodiment comprises an inserting-position information collecting unit, instead of the inserting-operation information collecting unit according to the first embodiment, and omits the endoscope video output unit because the fourth embodiment relates to the insertion of a blood vessel cure catheter (hereinafter, simply referred to a catheter) 51.

According to the fourth embodiment, a catheter inserting-length measuring unit 52 is arranged to detect an inserting position of the blood vessel cure catheter (hereinafter, simply referred to as the catheter) 51. Position information of a catheter distal-end portion which is detected by the measuring unit 52 is outputted to the inserting-position information collecting unit. The catheter inserting-length measuring unit 52 is arranged in an inner space portion of an apparatus main body 53 attached to the patient body surface. The apparatus main body 53 has opening portions 55a and 55b which can be inserted to the catheter at two positions of the distal-end side portion and the proximal end side portion.

Further, the apparatus main body 53 comprises: a guide wheel 62 which guides the catheter 51; and a pair of a first sandwiching wheel 61 and a second sandwiching wheel 64 to send or return the catheter. A gear 62 is coaxially attached to the second sandwiching wheel 64, and the gear 63 is rotated in accordance with the advance and return of the catheter 51 while the gear 63 sandwiches and presses the catheter 51. The gear 63 is geared to a worm gear 65, and a rotating shaft 66 of the worm gear 65 has an encoder 67, thus constituting the catheter inserting-length measuring unit 52.

A signal line 67a is extended from the encoder 67, and a signal from the encoder 67 is inputted via the signal line 67a to a catheter inserting-position processing device (not shown) having the same structure as that of the inserting-operation amount processing device 12 described according to the first embodiment. The inserting length and inserting position of the catheter 51 in a blood vessel 68 are calculated.

The operation of the catheter inserting-length measuring unit 52 with the above structure will be described.

The catheter 51 is guided to the outside of the apparatus main body 53 via the opening portion 55a formed at the distal-end portion of the apparatus main body 53. Then, the catheter 51 is guided to the inner space portion of the apparatus main body 53 again via the opening portion 55b formed at the proximal end portion. The catheter 51 is inserted between the first sandwiching wheel 61 and the second sandwiching wheel 64, and is pulled out/in by predetermined force. According to the fourth embodiment, the second sandwiching wheel 64 is rotated in accordance with the amount of insertion of the catheter 51 and, as a result of the rotation of the second sandwiching wheel 64, the gear portion 63 is rotated. Thus, the worm gear 65 geared to the gear portion 63 is rotated, the amount of rotation is converted into an electric signal by the encoder 67, and the inserting-length information is outputted to the catheter inserting-position processing device.

The catheter inserting-position processing device calculates the inserting-length of the catheter 51 in the blood vessel 68 based on the signal from the encoder 67, performs the processing similar to that of the first embodiment in accordance with the calculated inserting-length, and outputs to, displays at or notifies to the display device as the notifying unit of the instructing comment information to be displayed or notified, correlated with the inserting-position information.

With the above-mentioned structure and operation, substantially similar to the case of the endoscope inserting portion according to the first to third embodiments, the present invention can be applied to the blood vessel cure catheter 51 as the medical treatment tool having the long inserting portion.

According to the fourth embodiment, the blood vessel cure catheter 51 is described as the medical processing tool. However, the present invention is not limited to this and can be applied to an endoscope processing tool such as a clamp which is inserted in a channel for inserting an endoscope treatment tool.

(Fifth embodiment)

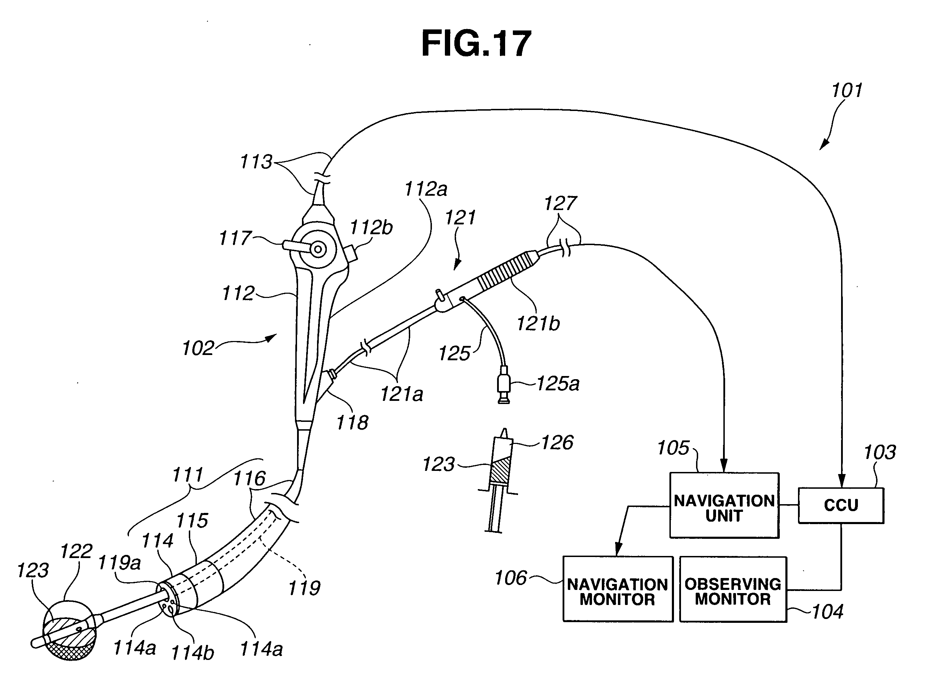

Figs. 17 to 22B show the fifth embodiment.

According to the fifth embodiment, the present invention is applied to a bronchoscope as an endoscope main body.

Referring to Fig. 17, an endoscope apparatus 101 according to the fifth embodiment mainly comprises: a bronchoscope (hereinafter, simply referred to as an endoscope) 102 as an endoscope main body which can be inserted to the bronchi; a camera control unit (hereinafter, referred to as a CCU) 103 which processes signals of an endoscope image obtained by the image pick-up operation of an image pick-up unit (not shown) in the endoscope 102; an observing monitor 104 which displays an endoscope observed image (hereinafter, referred to as an observed image) which is subjected to the signal processing by the CCU 103; a navigation unit 105 which forms a virtual endoscope image (hereinafter, referred to as a VBS image) that is generated based on a three-dimensional CT image preliminarily obtained by a CT device (not shown); and a navigation monitor 106 which displays the VBS image generated by the navigation unit 105.

The CT device is an X-ray CT (Computed Tomography) device which obtains three-dimensional image data of a sample by picking up a tomographic image of the sample and performs the diagnosis of the diseased part by using the three-dimensional image data.

The endoscope 102 comprises: a long inserting portion 111 with the flexibility; and an operating portion 112 which is connected to the proximal end side of the inserting portion 111 and which functions as a grip portion. A universal cord 113 is extended to the endoscope 102 from the back of the operation portion 112. A connector arranged to the end portion of the universal cord 113 is connected to the CCU 103.

The endoscope inserting portion 111 is formed by continuously connecting a distal-end portion 114 arranged to the distal-end side, a bending portion 115 arranged on the proximal end side of the distal-end portion 114, which can freely be bent, and a flexible tube portion 116 with the flexibility and the long dimension arranged on the proximal end side of the bending portion 115.

The endoscope operating portion 112 (operating portion 112 of the endoscope 102) has, on the proximal end side, a grip portion 112a as a portion which is gripped and held by the operator. The endoscope operating portion 112 has a video switch 112b for remotely controlling the CCU 103 on the top side of the grip portion 112a.

The endoscope operating portion 112 has a bending operating knob 117. The bending portion 115 is bent by gripping the grip portion 112a and rotating the bending operating knob 117.

Further, the endoscope operating portion 112 has a treatment tool inserting port 118 for inserting a treatment tool such as a biopsy clamp near the front end of the grip portion 112a. The treatment tool inserting port 118 is communicated to a treatment tool inserting channel 119. A treatment tool such as a biopsy clamp (not shown) is inserted in the treatment tool inserting port 118, the distal-end side of the treatment tool is projected from a channel opening 119a formed on a distal-end portion 114 via the treatment tool inserting channel 119 for the biopsy.

In the endoscope 102, a light guide (not shown) for transmitting illumination light is inserted and arranged to the inserting portion 111, operating portion 112, and universal cord 113. The proximal end side of the light guide reaches a connector portion of the universal cord 113 via the operating portion 112, and the light guide transmits illuminating light received from a light source (not shown). The illuminating light transmitted from the light guide illuminates a subject such as the diseased part from the distal-end surface of an illuminating window 114a fixed to the distal-end portion 114 of the inserting-portion.

An image of the illuminated subject is captured into the endoscope 102 from an observing window 114b arranged adjacently to the illuminating window 114a. The captured subject image is picked up, and is photoelectrically converted into an image pick-up signal by an image pick-up device (not shown). The image pick-up signal is transmitted to a signal cable (not shown), and is outputted to the CCU 103 via the universal cord 113.

The CCU 103 performs signal processing of the image pick-up signal from the image pick-up device of the endoscope 102, generates a standard video signal, outputs the video signal to an observing monitor 104, and displays an observed image on a display surface of the monitor 104.

As mentioned above, a navigation unit 105 forms the VBS image, outputs the generated video image to a navigation monitor 106, and displays the VBS image on the display surface of the navigation monitor 106 by linking to the position of the distal-end portion 114 of the endoscope inserting portion 111. The endoscope 102 is guided to the VBS image displayed on the navigation monitor 106 by the navigation unit 105, and the distal-end portion 114 of the inserting-portion reaches the interest portion in the bronchi.

The bronchi have multi-level-branches and further the observed images obtained at the branch points have the similar images having a plurality of branch routes.

Therefore, when the obtained observed image has a branch structure with a characteristic, the branch direction for the bronchi having the interest portion is easily distinguished. However, the branch direction having the interest portion is not distinguished based on only the image having no characteristics on the right and left branches of the obtained observed image.

According to the fifth embodiment, the endoscope apparatus 101 has a gravity direction instructing unit which visually instructs the gravity direction in accordance with the inclination of the endoscope 102, and has an arranging unit which arranges the gravity direction instructing unit within the range of field of view for observation of the endoscope 102.

That is, in the endoscope apparatus 101 according to the fifth embodiment, a probe 121 is inserted from the treatment tool inserting port 118 of the endoscope operation portion 112, the distal-end side of the probe 121 is projected from the channel opening 119a of the treatment tool inserting channel 119, and a balloon 122 as the gravity direction instructing unit arranged on the distal-end side thereof is arranged within the range of field of view for observation of the endoscope 102. That is, the probe 121 has the arranging unit which arranges the balloon 122 as the gravity direction instructing unit within the range of field of view for observation of the endoscope 102.

The probe 121 comprises a probe portion 121a which is inserted in the treatment tool inserting channel 119 of the endoscope 102, and a probe operating portion 121b which is arranged at the back end portion of the probe portion 121a.

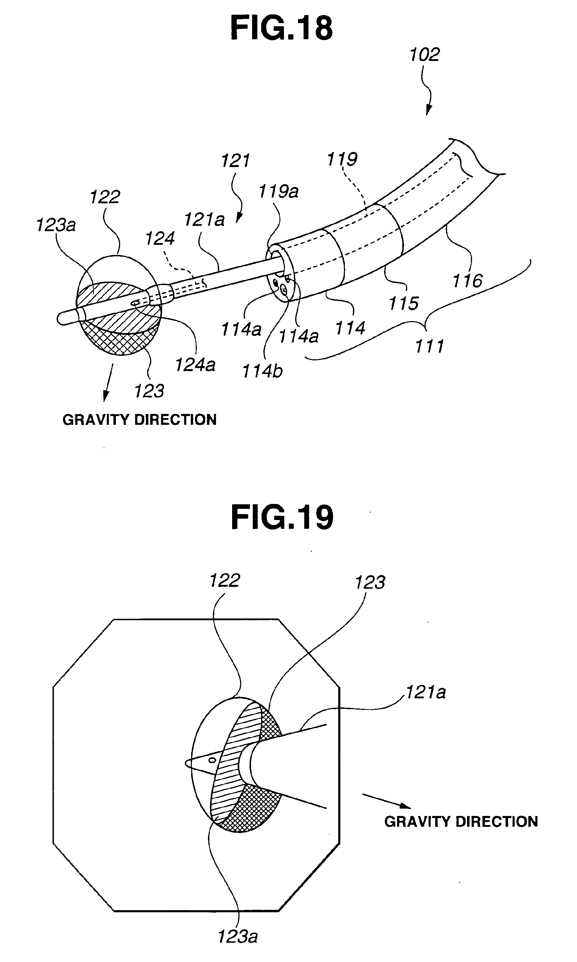

The probe portion 121a has the balloon 122 which is made of a transparent member or half-transparent member on the distal-end side, and a fluid tube 124 for supplying and discharging a fluid 123 is inserted and arranged into the balloon 122 through an opening 124a (refer to Fig. 18). An injecting cable 125 connected to the fluid tube 124 is extended to the probe operating portion 121b. The injecting cable 125 has an injecting cap 125a with a check valve at the end portion thereof. The probe 121 can supply and discharge the fluid 123 to a syringe 126 by connecting the syringe 126 to the injecting cap 125a with the check valve.

Few bronchi include a blue or green structure. Therefore, the fluid 123 is colored to blue or green. Thus, the fluid 123 can virtually be distinguished from the body organ such as the bronchi and further, advantageously, the range occupied by the blue or green is calculated by processing the image data.

The probes 121 is an ultrasonic probe which has an ultrasonic probe (not shown) at the probe distal-end portion and which obtains an ultrasonic tomographic image, and is connected to the navigation unit 105 via a probe cable 127.

The navigation unit 105 calculates the gravity direction of the distal-end portion 114 of the endoscope inserting portion 111 based on the observed image obtained by the image pick-up device of the endoscope 102, which will be described later, and performs processing for matching the rotating direction of the VBS image for the calculated gravity direction with the real image of a bronchoscope. That is, the navigation unit 105 constitutes an image processing unit. This processing may automatically be executed or may manually be executed by providing an instructing switch for this processing. When the probe 121 is an ultrasonic probe, the navigation unit 105 also performs, based on the gravity direction, the processing with respect to the ultrasonic tomographic image obtained by the ultrasonic probe, for matching the real image of the bronchoscope with the rotating direction.

The endoscope apparatus 101 with the above structure is used for the endoscope observation and processing (biopsy and cure) of a bronchi disease and the like.

First, the operator orally or nasally inserts the inserting portion 111 of the endoscope 102 in the body cavity of the patient, advances the distal-end portion 114 of the inserting-portion to a predetermined position which is determined by the operator, e.g., to the top end of the bronchus (laryngeal portion). Then, the operator inserts the endoscope inserting portion 111 while viewing the observed image obtained by the endoscope 102 displayed on the observing monitor 104.

The operator advances the distal-end portion 114 of the inserting-portion to the predetermined position, then, moves the navigation unit 105, refers to the VBS image displayed on the navigation monitor 106, and inserts the distal-end portion 114 of the inserting-portion in accordance with a route reaching the interest portion.

Here, as mentioned above, the bronchi have multi-level-branches and further the observed images obtained at the branch point has the similar images having a plurality of branching destination routes.

Thus, when the obtained observed image has the branch structure with the characteristic, the branch direction of the bronchi having the interest portion is easily distinguished. However, the branch direction having the interest portion is not distinguished only from the obtained observed image at the right and left branch without characteristic. On the contrary, when the gravity direction is determined, the branch direction is determined by comparison with the VBS image.

The operator inserts the probe 121 from the treatment tool inserting port 118 of the endoscope operating portion 112, and the distal-end side of the probe 121 is projected from the channel opening 119a of the treatment tool inserting channel 119. Incidentally, the probe 121 is not swollen yet.

The operator connects the syringe 126 to the injecting cap 125a with the check valve of the injecting cable 125, injects the fluid (liquid) 123 colored to blue or green with a predetermined amount in the balloon 122 of the probe portion 121a, injects the air, and thus swells the balloon 122. The two fluids (liquids) 123 with different colors and different specific gravity may be injected to the balloon 122 in place of injecting the fluid (liquid) 123 and the air. Further, the fluid 123 in use is a liquid according to the fifth embodiment. However, the present invention is not limited to this and may be a material like particles as the fluid 123.

Then, referring to Fig. 18, in the balloon 122, the fluid 123 colored to blue or green is sealed. The fluid 123 in the balloon 122 moves therein in accordance with the inclination of the distal-end portion 114 of the inserting-portion, and a fluid surface 123a changes in accordance with the gravity direction. Namely, on the fluid surface 123a, the vertical direction represents the gravity direction.

Thus, the balloon 122 can visually instruct the gravity direction. For example, referring to Fig. 19, upon obtaining the observed image, the fluid surface 123a of the fluid (liquid) 123 is inclined on the right and, therefore, it is determined that the gravity direction is approximately on the lower right.

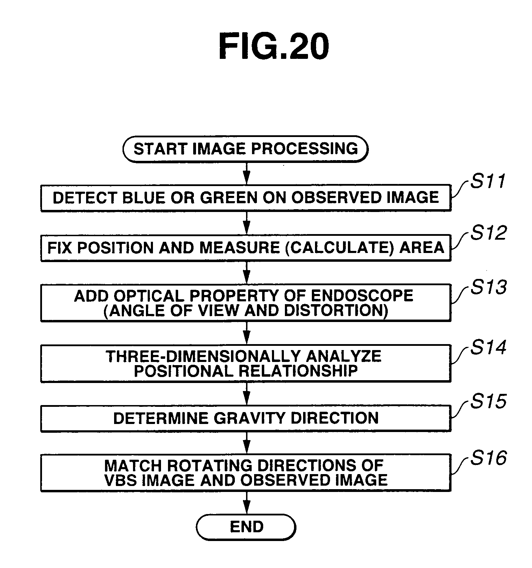

According to the fifth embodiment, the gravity direction of the distal-end portion 114 of the endoscope inserting portion 111 is calculated based on the observed image. The image processing is performed such that the rotating direction of the VBS image matches the calculated gravity direction. The navigation unit 105, for example, executes the image processing in accordance with a flowchart shown in Fig. 20.

The navigation unit 105 detects blue or green of the fluid 123 on the observed image obtained by the CCU 103 (step S11). The navigation unit 105 identifies the position or shape (pattern) of the portion occupied by the detected blue or green, and measures the area of the fluid surface 123a (step S12). Then, the navigation unit 105 adds the optical characteristics such as an angle of view or distortion of the endoscope 102 in use, and corrects the obtained positional relationship (step S13).

Next, the navigation unit 105 three-dimensionally analyzes the obtained positional relationship (step S14). The navigation unit 105 determines the gravity direction based on the obtained analysis result (step S15). The navigation unit 105 matches the rotating direction of VBS image with the rotating direction of the observed image based on the determined gravity direction (step S16).

Then, the navigation monitor 106 displays the VBS image in the direction matching that of the observed image. The operator bends the distal-end portion 114 of the inserting-portion to the peripheral part of the branched bronchi by using the bending portion 115 every branch, and the distal-end portion 114 of the inserting-portion reaches the interest portion. The operator executes the endoscope observation and treatment (biopsy and cure) of the interest portion.

Thus, the endoscope apparatus 101 according to the fifth embodiment can easily detect the gravity direction by the distal-end portion 114 of the inserting-portion, and the operator can prevent the missing of the gravity direction of the observed image upon inserting the inserting portion 111 into the complicated lumen.

As a result, the endoscope apparatus 101 according to the fifth embodiment can detect the gravity direction of the observed image and the operability is improved.

For example, in the endoscope apparatus 101, upon inserting the inserting portion 111 in the large intestine, the direction of the operating portion 112 is turned so as to easily insert the inserting portion 111 and, then, the gravity direction of the observed image can be known. Therefore, in the endoscope apparatus 101, it is known in which direction, the cancer exists when the affected area such as the cancer is found. Further, the operability is improved upon estimating into which organ the cancer infiltrates.

Further, when any crack is found in the inspection of a device or facilities having a pipe such as a plurality of pipes for heat exchange, in the endoscope apparatus 101, the gravity direction of the observed image is known. Therefore, the direction of the crack in the pipe is known in the endoscope apparatus 101, and the operability is improved upon estimating another pipe having the possibility that the corrosion is caused by the steam shot from the crack portion.

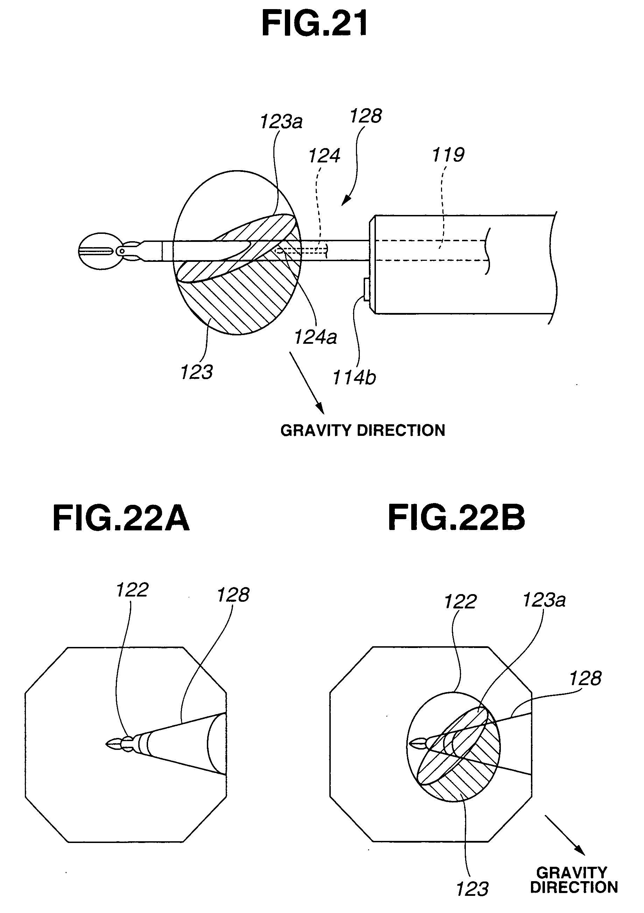

Instead of providing the probe 121, referring to Fig. 21, the balloon 122 may be arranged on the distal-end side of the treatment tool 128 such as a clamp. In this case, the observed images are obtained as shown in Fig. 22A or 22B.

Fig. 22A shows the observed image in a state before swelling the balloon 122 arranged to the distal-end side of the treatment tool 128, and Fig. 22B shows the observed image in a state after swelling the balloon 122 arranged on the distal-end side of the treatment tool 128 changing from the state shown in Fig. 22A.

The endoscope apparatus 101 according to the modification example can detect the gravity direction and can implement the treatment such as the biopsy and cure by arranging the balloon 122 on the distal-end side of the treatment tool 128.

That is, the endoscope apparatus 101 according to the modification example has a merit that after detecting the gravity direction, it promptly shifts to the treatment such as the biopsy or cure without pulling out the probe.

The endoscope apparatus comprises the electronic endoscope which picks up the endoscope image at the distal-end portion 114 of the inserting-portion thereof. However, the present invention is not limited to this. The present invention may be applied to an optical endoscope in which the endoscope image captured from the distal-end portion 114 of the inserting-portion is transmitted to an eye piece portion by an image transmitting unit and is observed by the eye piece portion.

(Sixth embodiment)

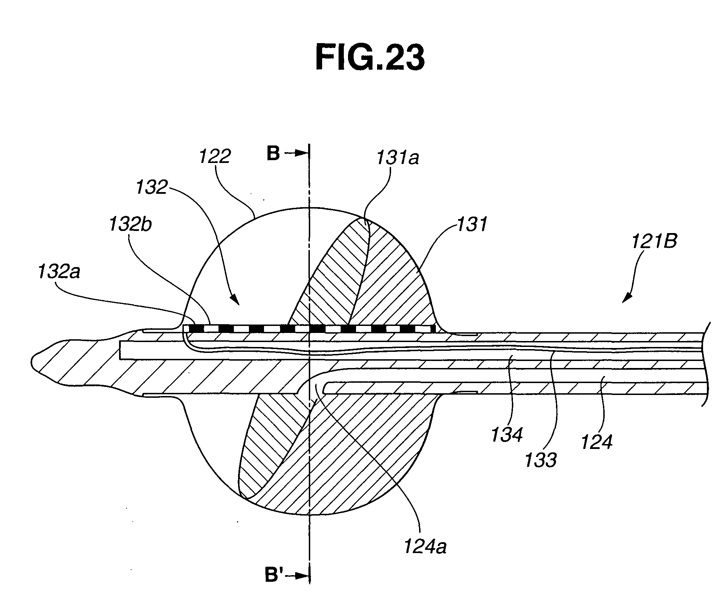

Figs. 23 to 28 are diagrams according to the sixth embodiment.

According to the sixth embodiment, the fluid 123 which is sealed in the balloon 122 is a conductive fluid. Other structures are the same as those according to the fifth embodiment, therefore, a description thereof is omitted, and the same reference numerals denote the same components.

That is, referring to Fig. 23, an endoscope apparatus according to the sixth embodiment comprises a probe 121B using a conductive fluid 131 as the fluid 123 which is sealed in the balloon 122. The fluid tube 124 inserted and arranged similarly to the fifth embodiment is connected to the injecting cable 125 in the probe operating portion 121b to supply and discharge the conductive fluid 131 to the balloon 122 from the syringe 126.

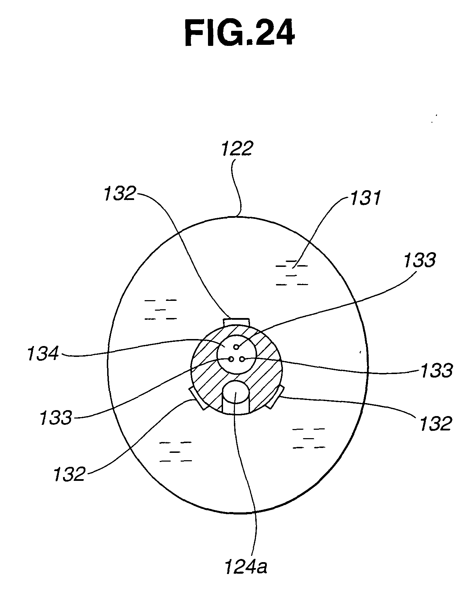

Referring to Fig. 24, the probe 121B has at least three electrodes 132 to be contact with the conductive fluid 131 in the balloon 122.

In the electrodes 132, conductive portions 132a and insulating portions 132b are alternately arranged. Electric wirings 133 for supplying current to the conductive portions 132a are inserted in electric wiring inserting passages 134 and are extended to the probe operating portion 121b.

By connecting the probe 121B to the navigation unit 105 via a probe cable 127, current is supplied to the electrodes 132 from the navigation unit 105.

According to the sixth embodiment, based on the current change of the electrodes 132, the positional change of the conductive fluid 131 is calculated and the gravity direction is determined.

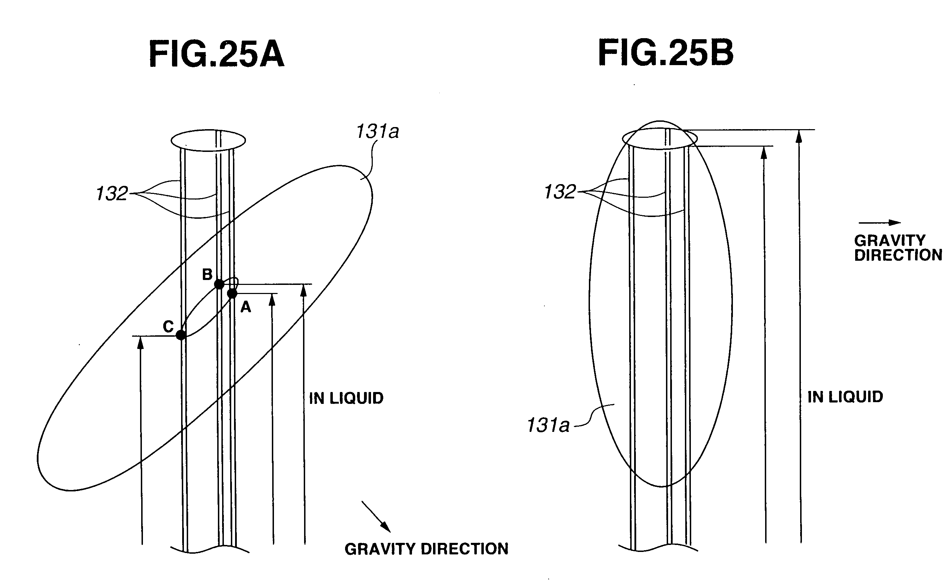

Referring to Fig. 25A, for example, when the fluid surface 131a of the conductive fluid 131 is inclined on the right, the three electrodes 132 are conductive on the side lower than A, B, and C points. The current flowing to the electrodes 132 changes depending on the conductive range. Based on the current change, the navigation unit 105 calculates the positional change of the conductive fluid 131 and determines that the gravity direction is on the lower right. Referring to Fig. 25B, for example, when the fluid surface 131a of the conductive fluid 131 is contact with all the three electrodes 132 and then they are conductive, the navigation unit 105 determines that the gravity direction is on the right.

Thus, the endoscope apparatus 101 according to the sixth embodiment has the same advantages as those according to the fifth embodiment.

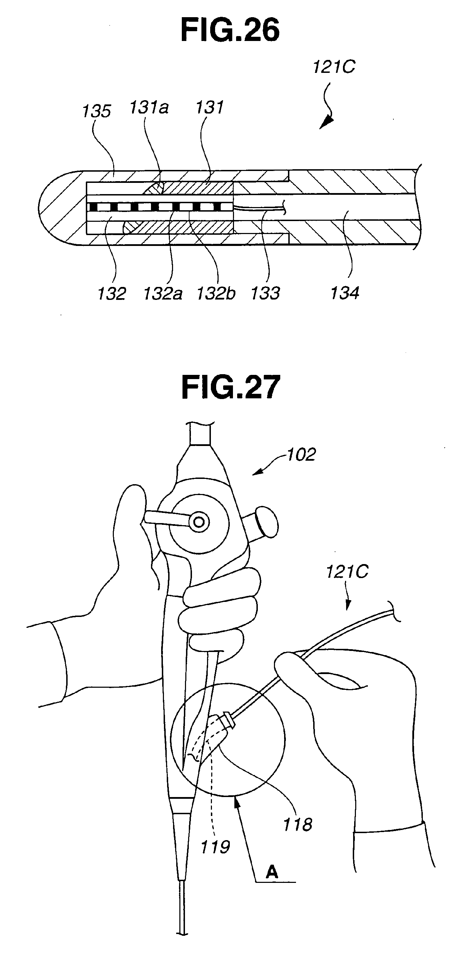

Referring to Fig. 26, the probe may seal the conductive fluid 131 without providing the balloon 122 on the distal-end side thereof.

Referring to Fig. 26 again, a probe 121C has a fluid sealing portion 135 which seals the conductive fluid 131, instead of the balloon 122 on the distal-end side, and further has at least the three electrodes 132 in the fluid sealing portion 135 similarly to the above description according to the sixth embodiment. The fluid sealing portion 135 is watertightly formed so as to prevent the leakage of the conductive fluid 131 to the electric wiring inserting passage 134 in which the electric wiring 133 extended from the electrodes 132 is inserted.

The probe 121C does not need to be arranged within the range of field of view for observation of the endoscope 102. The probe 121C is inserted in the distal-end portion 114 of the endoscope inserting portion 111 and in the state the positional change of the conductive fluid 131 can be calculated and the gravity direction can be determined based on the change in the current flowing the three electrodes 132 in the same way as that in the above description according to the sixth embodiment.

The probe 121C needs the correction of the rotating direction of the endoscope 102. In this case, referring to Fig. 27, the probe 121C is inserted in the treatment tool inserting channel 119 from the treatment tool inserting port 118 of the endoscope 102.

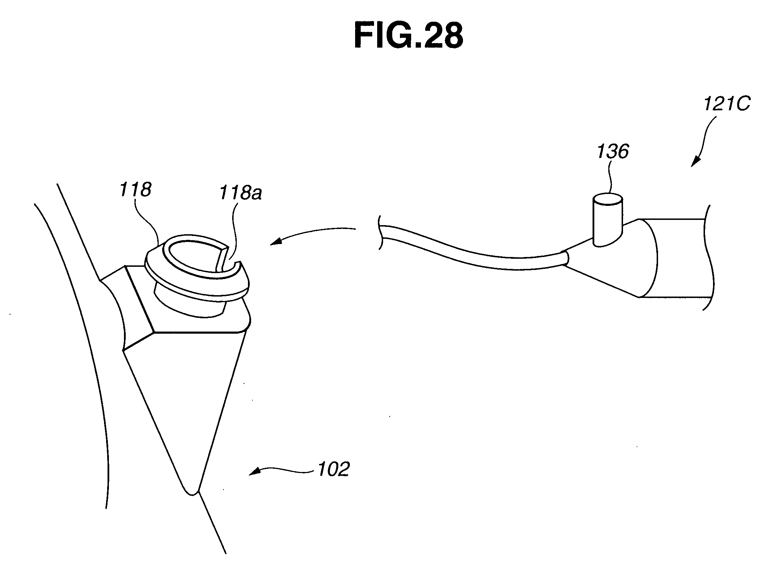

Referring to Fig. 28, for the purpose of correcting the rotating direction of the endoscope 102, in the probe 121C, a projected portion 136 arranged on the proximal end side of the probe portion 121a is engaged with a notch 118a formed on the treatment tool inserting port 118 of the endoscope 102. Thus, the probe 121C can correct the rotating direction of the endoscope 102.

The structure according to the modification has the same advantages as those according to the sixth embodiment. In addition, the diameter can be shorter because the balloon 122 is not necessary and, for example, the endoscope apparatus can reach the deepest portion of the bronchus.

(Seventh embodiment)

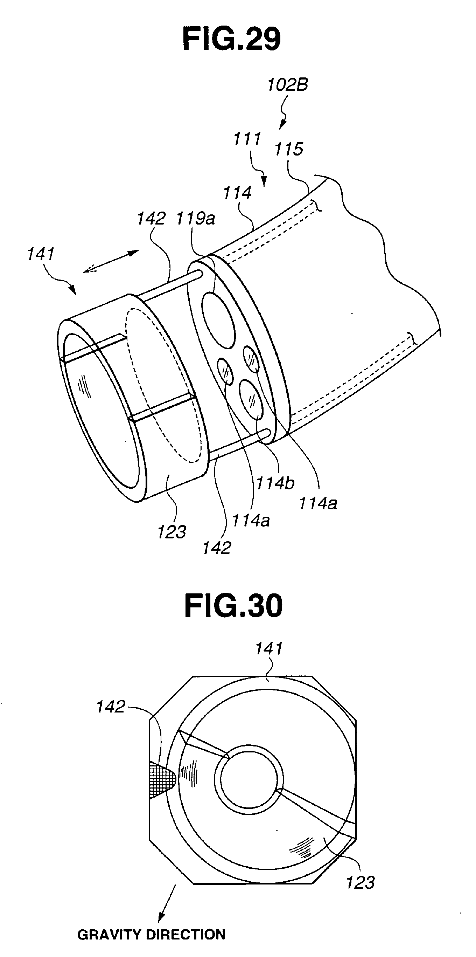

Figs. 29 to 34 are diagrams according to the seventh embodiment.

The gravity direction is detected by using the probe according to the fourth and sixth embodiments. However, the gravity direction is detected by providing the fluid sealing portion at the distal-end portion 114 of the endoscope inserting portion 111. Other structures are the same as those according to the fifth embodiment, therefore, a description thereof is omitted, and the same reference numerals denote the same components.

That is, referring to Fig. 29, an endoscope 102B according to the seventh embodiment has a fluid sealing portion 141 for detecting the gravity direction at the distal-end portion 114 of the inserting-portion.

The fluid sealing portion 141 is a transparent member or semi-transparent member, and is formed to be hollow with the same outer diameter as that of the distal-end portion 114 of the inserting-portion. Further, the fluid sealing portion 141 has an exterior member which seals the fluid (liquid) 123 and air similar to that according to the fifth embodiment, or two fluids (liquids) 123 with different densities. In the fluid sealing portion 141, the hollow portion becomes an observed-window area.

Furthermore, the fluid sealing portion 141 presses a rod member 142 which is inserted in the inserting portion 111, thereby freely advancing or returning the fluid sealing portion 141 in the longitudinal direction of the inserting portion 111. The rod member 142 is pressed or pulled by an advancing operating mechanism (not shown) arranged in the operating portion 112.

Upon detecting the gravity direction in the endoscope 102B, the rod member 142 is pressed in the direction opposite to the distal-end portion 114 of the inserting-portion, and thus the fluid sealing portion 141 enters the range of field of view for observation. Referring to Fig. 30, the endoscope 102B obtains an observed image. In the observed image shown in Fig. 30, since the fluid surface 123a of the fluid (liquid) sealed in the fluid sealing portion 141 is inclined on the diagonal left, it is determined that the gravity direction is approximately on the lower left.

Similarly to the fifth embodiment, the navigation unit 105 calculates the gravity direction of the distal-end portion 114 of the endoscope inserting portion 111 based on the observed image, and performs the image processing in which the rotating direction of the VBS image matches the calculated gravity direction.

Upon observation except for detecting the gravity direction, in the endoscope 102B, the rod member 142 is pulled to the side of the distal-end portion 114 of the inserting-portion. Thus, the fluid sealing portion 141 is close to the distal-end portion 114 of the inserting-portion and the field of view over the observed window area is obtained.

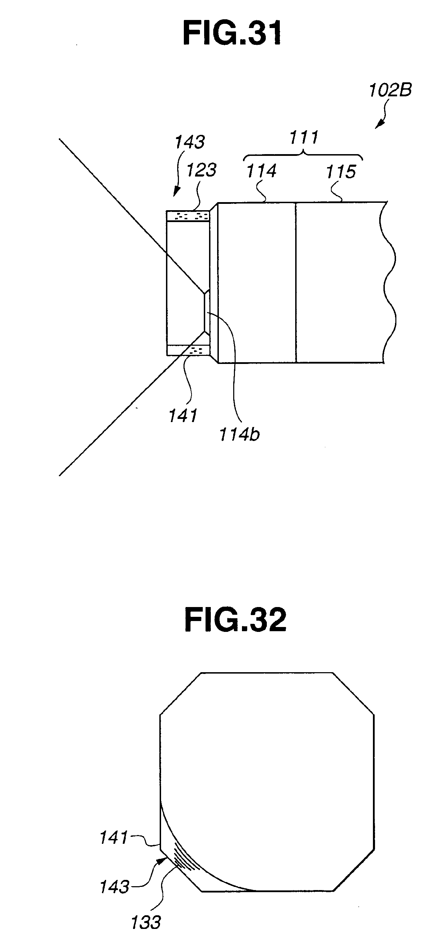

Referring to Fig. 32, the endoscope 102B appears only at the peripheral portion of the observed image.

As a result, the endoscope 102B according to the seventh embodiment has the same advantages as those according to the fifth embodiment and the operability is improved because the probe is not used.

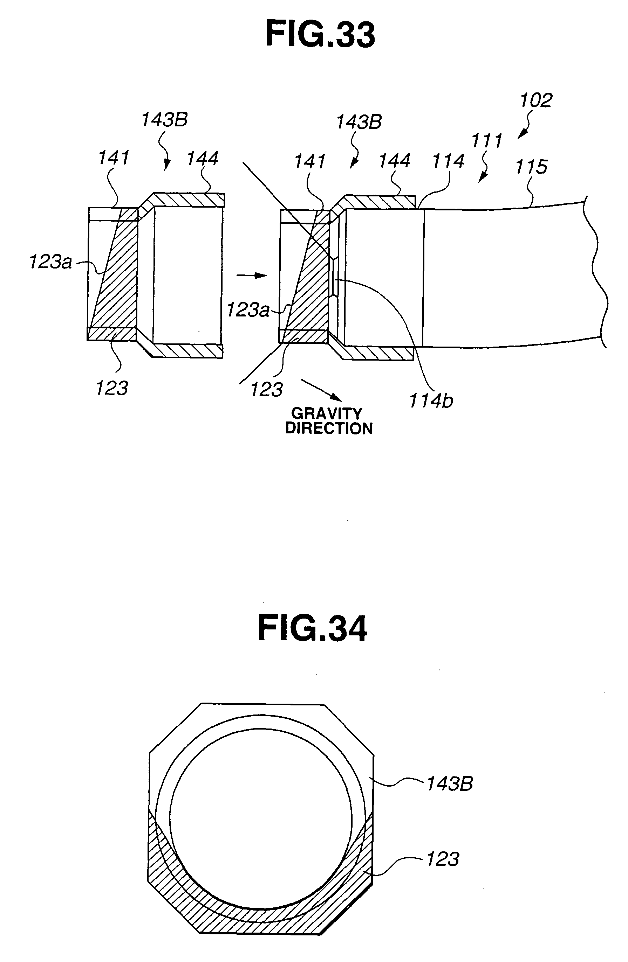

Referring to Fig. 33, the endoscope does not have the fluid sealing portion 141 in the distal-end portion 114 of the inserting-portion thereof and a cap 143B may have the fluid sealing portion 141.

That is, referring to Fig. 33, the endoscope 102 is a transparent member or semi-transparent member, and has the cap 143B which is formed with the same outer diameter as that of the distal-end portion 114 of the inserting-portion.

That is, referring to Fig. 33 again, the cap 143B has the same fluid sealing portion 141 as that according to the seventh embodiment on the distal-end side, and an attaching portion 144 with which the distal-end side of the endoscope inserting portion 111 is engaged on the proximal end side thereof.

The endoscope 102 with the above structure obtains an observed image as shown in Fig. 34. Referring to Fig. 34, the fluid 123 sealed in the fluid sealing portion 141 is inclined just to the bottom and therefore it is determined that the gravity direction is on the bottom. Referring to Fig. 33, the fluid surface 123a is inclined on the diagonal right in the observed image, the gravity direction is approximately on the lower right.

Similarly to the fifth embodiment, the navigation unit 105 calculates the gravity direction of the distal-end portion 114 of the endoscope inserting portion 111, and performs the image processing in which the rotating direction of the VBS image matches the calculated gravity direction.

As a result, the endoscope according to the modification has the same advantages as those according to the seventh embodiment and the rod member 142 of the fluid sealing portion 141 is not necessary. Thus, the diameter can be shorter.

(Eighth embodiment)

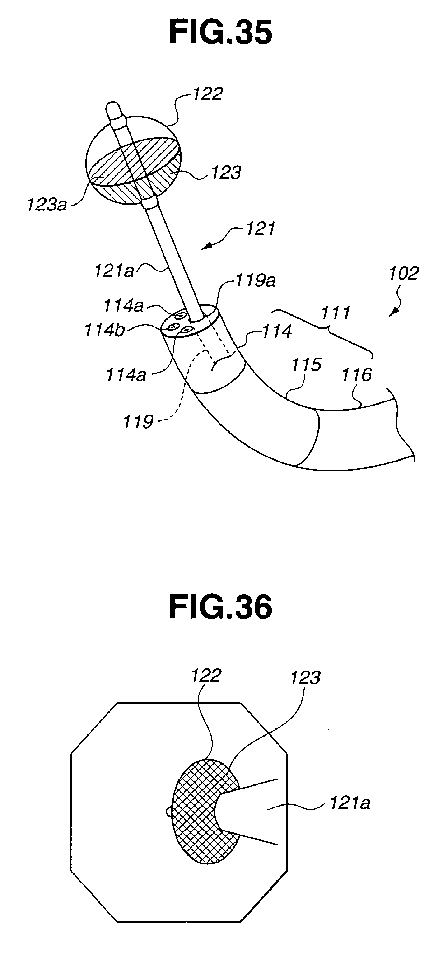

Figs. 35 to 44 are diagrams according to the eighth embodiment.

In the lumen in the body cavity is bent in the endoscope apparatus according to the fifth embodiment, when the balloon 122 is, for example, projected in the upper diagonal direction from the distal-end portion of the inserting-portion of the endoscope which is in the upper diagonal direction as shown in Fig. 35, the observed image obtained from the distal-end portion of the inserting-portion becomes an image which is viewed from the bottom.

In this case, referring to Fig. 36, the fluid surface 123a of the fluid 123 sealed in the balloon 122 is not viewed and therefore the gravity direction in the observed image is not determined.

According to the eighth embodiment, in addition to the fluid 123, at least two spherical members with different densities are sealed in the balloon 122 and the gravity direction is detected. Other structures are the same as those according to the fifth embodiment, therefore, a description thereof is omitted, and the same reference numerals denote the same components.

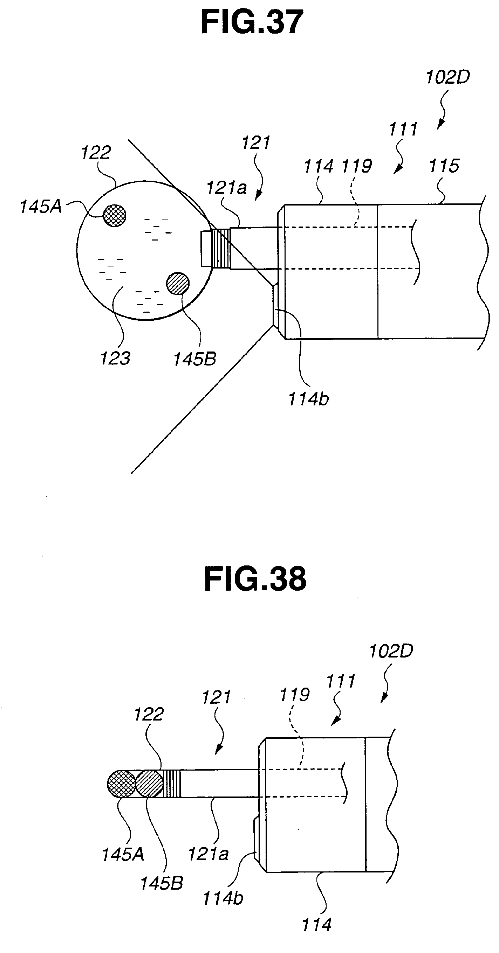

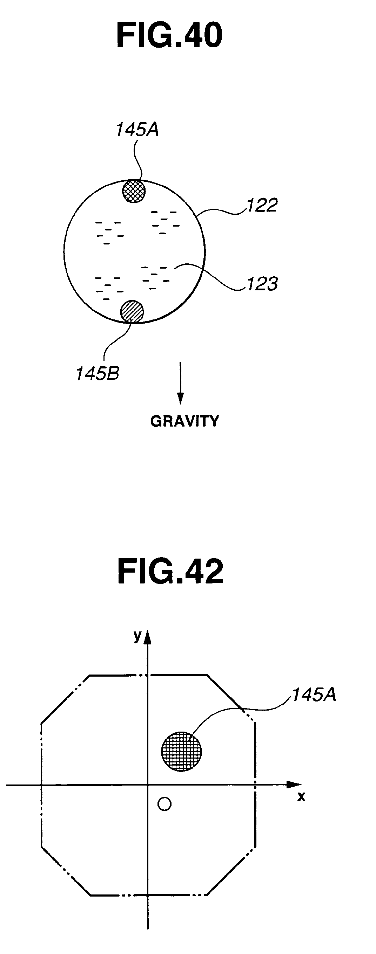

Referring to Fig. 37, in an endoscope 102D according to the eighth embodiment, the fluid 123 and at least two spherical members 145A and 145B with different densities are sealed in the balloon 122 arranged to the probe 121.

Among the two spherical members 145A and 145B, the spherical member 145A is colored to green and the spherical member 145B is colored to blue. The densities of the spherical members 145A and 145B have a relationship, for example, of spherical member 145A (green) < fluid 123 < spherical member 145B (blue).

Similarly to the fifth embodiment, the fluid 123 is supplied and discharged from the syringe 126 to the balloon 122. In the contracting state of the balloon 122 before supplying the fluid 123 (swelling), referring to Fig. 38, the contraction force of the balloon member arranges and accommodates the two spherical members 145A and 145B on the axis of the probe 121C on the distal-end side thereof.

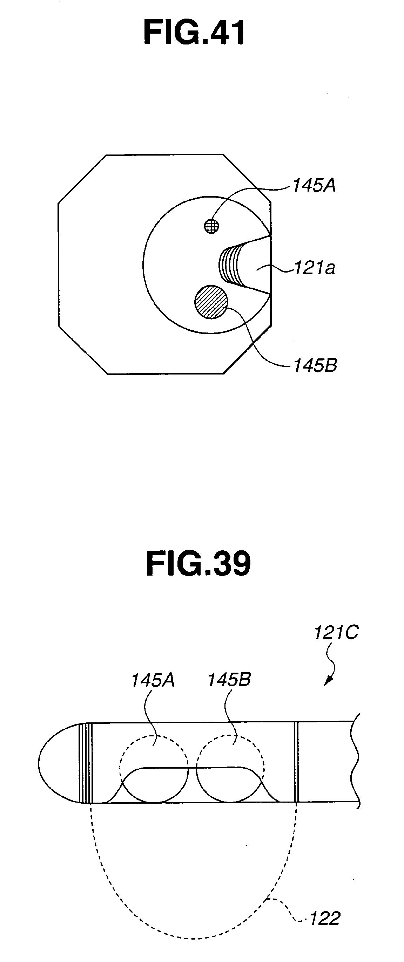

Referring to Fig. 39, the balloon 122 may be arranged such that the two spherical members 145A and 145B are accommodated in the distal-end side of the probe 121C.

Then, referring to Fig. 40, the fluid 123 is supplied to the balloon 122 and thus the balloon 122 swells.

With the above-mentioned density relationship, the density of the spherical member 145A (green) is lighter than that of the fluid 123. Thus, the spherical member 145A (green) floats on the fluid 123. On the other hand, the spherical member 145B (blue) sinks on the bottom of the fluid 123 because the density of the spherical member 145B (blue) is heavier than that of the fluid 123. Consequently, it is determined that the gravity direction is on the direction of the spherical member 145B (blue) on the straight line passing through the center of the spherical member 145A (green) and the center of the spherical member 145B (blue). For example, referring to Fig. 40, the gravity direction is the bottom direction.

With the endoscope 102D having the above structure has the observed image as shown in Fig. 41 in a state in which the balloon 122 is projected in the upper diagonal direction from the distal-end portion 114 of the inserting-portion of the endoscope in the upper diagonal direction as shown in Fig. 35.

Similarly to the fifth embodiment, the navigation unit 105 calculates the gravity direction of the distal-end portion 114 of the endoscope inserting portion 111 based on the observed image, and performs the image processing in which the rotating direction of the VBS image matches the calculated gravity direction.

With the above-mentioned density relationship, referring to Fig. 41, the spherical member 145A (green) is viewed to be small at the far position because it floats on the fluid 123. On the other hand, the spherical member 145B (blue) is viewed to be large at the close position because the fluid 123 sinks on the bottom of the fluid 123.

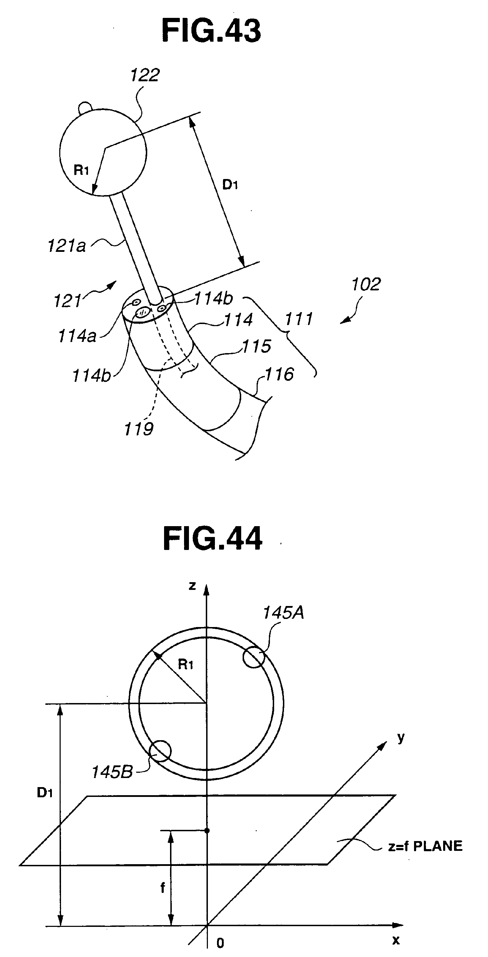

Therefore, referring to Fig. 42, the ranges (sizes) and the positions of the spherical members 145A (green) and 145B (blue) occupying on the image need to be recognized and the three-dimensional positional relationship among the spherical member 145A (green) and the spherical member 145B (blue) needs to be derived.

Next, a description is given of the three-dimensional positional relationship.

First, a state in which the balloon 122 is projected from the distal-end portion 114 of the endoscope inserting portion 111 is considered in a model shown in Fig. 43 for the sake of a brief description.

Here,

Origin O: Center of view point of the endoscope 102D

R2: Radiuses of the spherical members 145A and 145B

R1: Radius of the balloon 122 (R1 >> R2)

f: Focusing distance

D1: Center distance between the balloon 122 and the distal-end portion 114 of the endoscope inserting portion 111.

The spherical members 145A and 145B receive respectively the gravity and thus move in the balloon 122. Then, the centers of the spherical members 145A and 145B move on the spherical surface with the radius of (R1 - R2).

However, a relationship of (R1 >> R2) is established and thus the radius is approximate to R1.

That is, the centers of the spherical members 145A and 145B are as follows.

$$x^2 + y^2 + (z - D1)^2 = R1^2 \qquad (1)$$

The center $O_A$ (X, Y, Z) of the spherical member 145A is considered as follows based on the formula (1)

$$(Z > D1) \qquad (2)$$

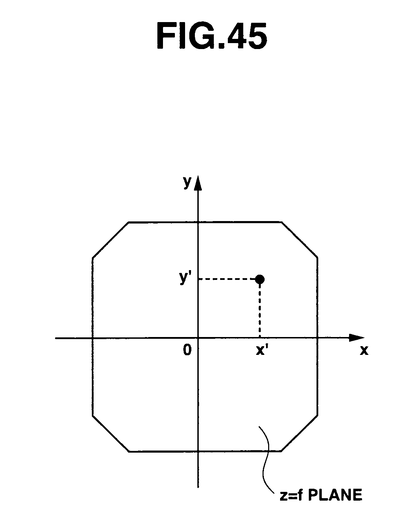

On the observed image, the subject is projected with a focusing distance f and the formula (2) is considered in the case of the projection on z = f plane as shown in Fig. 45.

It is assumed that the coordinate after the projection is (x', y', z').

$$x' : X = z' : Z \qquad (3)$$

$$y' : Y = z' : Z \qquad (4)$$

Based on the formulae (2), (3), and (4),

$$\qquad (5)$$

$$\qquad (6)$$

That is, referring to Fig. 45, the x and y coordinates (x', y') on the z = f plane are measured based on the observed image and thus the x and y coordinates (X, Y) of the spherical member 145A are obtained based on the formulae (5) and (6) and the z coordinate is obtained based on the formula (2) (if the center coordinates are read based on the observed image, the three-dimensional coordinates are determined).

As mentioned above, the coordinates of the spherical member 145A are determined on the three-dimensional coordinate system.

Since the spherical member 145B has the target center (0, 0, D1), the coordinates of the spherical member 145B is (-X, -Y, 2D1 - Z)

As mentioned above, a vector AB in the gravity direction is as follows.

$$\text{Vector AB} = (0-X, \ 0-Y, \ D1 - Z)$$

$$= (-X, \ -Y, \ D1 - Z)$$

The sizes of the spherical members 145A and 145B are measured on the observed image for the purpose of determining which of the spherical members 145A and 145B is closer to the view point (the size is measured but the distance is not calculated).

When the spherical members 145A and 145B have the some-extent size (occupying any desired range on the image), the distortion increases as the spherical members 145A and 145B are more apart from the center of the observed image. Therefore, upon reading the center coordinates (x', y') of the spherical members 145A and 145B, the correction is necessary. The basic calculating method of the three-dimensional coordinates is the same as that of the foregoing.

Thus, the endoscope 102D according to the eighth embodiment can derive the three-dimensional positional relationship between the spherical members 145A (green) and 145B (blue). When the spherical member 145 in the balloon 122 has the some-extent size and it is determined based on the observed image, on which of the top and the bottom of the z = D1 plane, the center of the spherical member 145 is, the same advantages are obtained when the spherical member 145 in the balloon 122 is one.

As a result, the endoscope 102D according to the eighth embodiment has the same advantages as those according to the fifth embodiment. When the lumen in the body cavity is bent, the gravity direction is easily determined when the balloon 122 is projected in the upper diagonal direction from the distal-end portion 114 of the inserting-portion of the endoscope in the upper diagonal direction.

(Ninth embodiment)

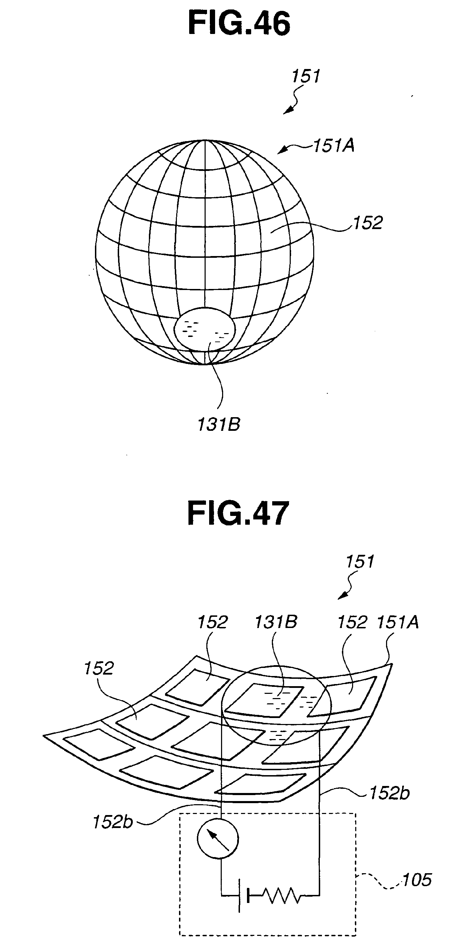

Figs. 46 and 47 are diagrams according to the ninth embodiment.

According to the ninth embodiment, a gravity sensor is used with the arrangement to the endoscope inserting portion, probe, or the distal-end side of the treatment tool. Other structures are the same as those according to the fifth embodiment, a description thereof is omitted, and the same reference numerals denote the same components.

That is, referring to Fig. 46, according to the ninth embodiment, the endoscope has a gravity sensor 151 as a gravity direction instructing unit arranged on the distal-end side of the endoscope inserting portion, probe, or treatment tool. The gravity sensor 151 has a plurality of minute electrodes 152 in a spherical container 151A. Liquid drops 131B of a conductive fluid 131 with low wettability which move in accordance with the gravity direction on the minute electrodes 152 are sealed in the spherical container 151A. A signal line 152b extended from the minute electrodes 152 is electrically connected to the navigation unit 105 and the minute electrodes 152 are controlled by the navigation unit 105.

Referring to Fig. 47, the navigation unit 105 spherically scans the gravity sensor 151 and the resistance is measured between the adjacent minute electrodes 152, thereby detecting the presence of the liquid drops 131B at the portion having the low resistance. Based on the detected position, the gravity direction is calculated.

In the gravity sensor 151, the spherical container 151A may be filled with the conductive fluid 131 and the gravity direction may be detected based on bubbles moving in the conductive fluid 131. In this case, the navigation unit 105 spherically scans the gravity sensor 151 and measures the resistance between the adjacent minute electrodes 152, thereby detecting the bubbles at the portion having the high resistance. Based on the detecting position, the gravity direction is calculated.

Thus, as compared with the fourth to eighth embodiments, the endoscope according to the ninth embodiment has the distal-end portion 114 of the inserting-portion whose diameter can be shorter.

Having described the embodiments of the present invention, it should be understood that the present invention is not limited to those specific embodiments and various changes and modifications thereof could be made without departing from the spirit or scope of the present invention as defined in the appended claims.

Industrial Applicability

As mentioned above, according to the present invention, the endoscope system, inserting operation program of the endoscope inserting portion, and endoscope apparatus are useful for the medical observation of the body cavity and various cures and treatments and further are suitable for medical education. Further, according to the present invention, the endoscope apparatus is useful for inspecting the scratch and corrosion of a tube or tank of various facilities, fuselage or wing of an aircraft, piping of a boiler, gas turbine, and chemical plant, and body of an automobile engine and the like, as well as the medical use.

Cross-reference of related applications

The present application is filed based on claiming priority of Japanese Patent Application 2002-255696 filed to Japan on the 30th August, 2002, and Japanese Patent Application 2002-255700 filed to Japan on the 7th November, 2002. The disclosure contents are referred to in the description, claims, and drawings of the present application.

**Claims**

**1.** A medical treatment system having a long inserting portion which is inserted in a sample, the medical treatment system comprising:

a positional relationship detecting unit which detects a relative positional relationship between the sample and a distal-end portion of the inserting portion;

an information input unit which can input predetermined information; and

a storing unit which stores the predetermined information and the positional information detected by the positional relationship detecting unit with a correlation therebetween.

2. An endoscope system having an inserting portion which is inserted in a sample, the endoscope system comprising:

a positional relationship detecting unit which detects a relative positional relationship between the sample and a distal-end portion of the inserting portion;

an information input unit which can input predetermined information; and

a storing unit which stores the predetermined information and the positional information detected by the positional relationship detecting unit with a correlation therebetween.

3. An endoscope system according to Claim 2, wherein the positional relationship detecting unit is an inserting-length detecting unit which detects the inserting length of the sample in the inserting portion of the endoscope.

4. An endoscope system according to Claim 3, wherein the positional relationship detecting unit has at least one of a bending angle detecting unit which detects a bending angle of a bending portion of the endoscope and a turn angle detecting unit which detects a turn angle of the inserting portion of the endoscope.

5. An endoscope system according to Claim 2, wherein the predetermined information is at least one of endoscope image information in the sample picked up by the endoscope, character information, a virtual endoscope image generated based on three-dimensional data of the sample, and inserting-operation information.

6. An endoscope inserting-operation program for inserting an endoscope inserting portion in a sample, the endoscope inserting-operation program comprising:

a positional relationship detecting step for detecting a relative positional relationship between the sample and a distal-end portion of the inserting portion;

an information input step for inputting predetermined information; and

a storing step for storing the predetermined information and the positional information detected by the positional relationship detecting step with a correlation therebetween.

7. An endoscope inserting-operation program according to Claim 6, wherein the positional relationship detecting step is an inserting-length detecting step which detects the inserting length of the sample in the inserting portion of the endoscope.

8. An endoscope inserting-operation program according to Claim 7, wherein the positional relationship detecting step has at least one of a bending angle detecting step which detects a bending angle of a bending portion of the endoscope and a turn angle detecting step which detects a turn angle of the inserting portion of the endoscope.

9. An endoscope inserting-operation program according to Claim 6, wherein the predetermined information is at least one of endoscope image information in the sample picked up by the endoscope, character information, a virtual endoscope image generated based on three-dimensional data of the sample, and inserting-operation information.

10. An endoscope system having an inserting portion which is inserted in a sample, the endoscope system comprising:

a storing unit which stores predetermined information which is previously correlated with relative positional information between the sample and a distal-end portion of the inserting portion;

a positional relationship detecting unit which detects a relative positional relationship between the sample and the distal-end portion of the inserting portion; and

an information output unit which can output, from the storing portion, predetermined information which is correlated with the positional relationship information detected by the positional relationship detecting unit.

11. An endoscope system according to Claim 10, further comprising:

a driving unit which performs the operation for inserting the inserting portion in the sample based on the predetermined information outputted from the information output unit.

**12.** An endoscope system according to Claim 10, wherein the predetermined information is at least one of image information in the sample picked up by the endoscope, character information, an virtual image generated based on three-dimensional data of the sample, and inserting-operation information.

**13.** An endoscope inserting-operation program for inserting an endoscope inserting portion in a sample, the endoscope inserting-operation program comprising:

a positional relationship detecting step for detecting a relative positional relationship between the sample and a distal-end portion of the inserting portion; and

an information output step for outputting predetermined information corresponding to positional information detected by the positional relationship detecting step, from a storing unit which previously stores predetermined information that is correlated with the relative positional relationship between the sample and the distal-end portion of the inserting portion.

**14.** An endoscope inserting-operation program according to Claim 13, wherein the positional relationship detecting step is an inserting-length detecting step for detecting the inserting length of the sample in the inserting portion of the endoscope.

**15.** An endoscope inserting-operation program according to Claim 14, wherein the positional relationship detecting step has at least one of a bending angle detecting step which detects a bending angle of a bending portion of the endoscope and a turn angle detecting step which detects a turn angle of the inserting portion of the endoscope.

**16.** An endoscope inserting-operation program according to Claim 13, wherein the predetermined information is at least one of endoscope image information in the sample picked up by the endoscope, character information, a virtual image generated based on three-dimensional data of the sample, and inserting-operation information.

**17.** An endoscope apparatus comprising:

a detecting unit which detects inserting-operation information of an endoscope inserting portion which is inserted in a sample;

a storing unit which stores standard inserting-operation information that is detected by the detecting unit and an endoscope image obtained by picking up an image of the sample at the position of a distal-end portion of the endoscope inserting portion upon the inserting operation with a correlation therebetween; and

a control unit which compares the standard inserting-operation information stored in the storing unit with the inserting-operation information obtained from the detecting unit during the operation and which monitors the inserting operation situation of the endoscope inserting portion.

**18.** An endoscope apparatus according to Claim 17, wherein the inserting-operation information is at least one of inserting-length information for measuring the inserting length of the endoscope inserting portion, inserting speed information for measuring the inserting speed of the inserting portion, turn angle information for measuring a turn angle of the inserting portion, and angle information for measuring an angle of a bending portion of the inserting portion.

**19.** An endoscope apparatus according to Claim 17, wherein the storing unit adds inserting-operation instructing comment information to a corresponding portion of the stored inserting-operation information and endoscope image information with the correlation therebetween, and stores it.

**20.** An endoscope apparatus according to Claim 17, wherein the control unit further compares the endoscope image at the position of the distal-end portion of the inserting portion upon the inserting operation, the endoscope image is stored in the storing unit correlated with the standard inserting-operation information, with the endoscope image at the position of the distal-end portion of the inserting portion upon the inserting operation during the operation, and monitors the situation of the endoscope inserting operation.

**21.** An endoscope apparatus according to Claim 17 or 20, further comprising:

a notifying unit which notifies of the inserting operation situation and an operating instruction based on a comparison and analysis result of the control unit.

**22.** An endoscope apparatus according to Claim 19, wherein the inserting operation instructing comment additionally stored in the storing unit is notified from the notifying unit based on the inserting length of the inserting portion which is detected by a detecting unit for detecting the inserting operation information.

**23.** An endoscope apparatus according to Claim 17, wherein the control unit sequentially analyzes the endoscope image obtained from the distal-end portion of the inserting portion and the inserting operation information obtained from the detecting unit by comparing with the standard inserting-operation information read from the storing unit and the endoscope image correlated with the operation information, outputs an analysis result to the notifying unit, monitors the endoscope inserting operation situation of the endoscope of the operator, and gives an instruction for the inserting operation.

**24.** An endoscope inserting-operation program for inserting an inserting portion of an endoscope in a sample, comprising:

a detecting step for detecting inserting-operation information of the endoscope inserting portion that is inserted in the sample;
a storing step for storing standard inserting-operation information that is detected by the detecting unit and an endoscope image obtained by picking up an image of the sample at the position of a distal-end portion of the endoscope inserting portion upon the inserting operation with a correlation between; and
a comparing and monitoring step for comparing the standard inserting-operation information stored in the storing step with the inserting-operation information obtained by the detecting step during the operation and of monitoring the situation of the inserting operation of the endoscope inserting portion.

**25.** An endoscope inserting-operation program according to Claim 24, further comprising:

an additionally recording step for adding inserting-operation instructing comment information to a corresponding portion of the stored inserting-operation information and endoscope image information with the correlation therebetween, and for storing it.

**26.** An endoscope inserting-operation program according to Claim 25, further comprising:

a notifying step for notifying of the inserting-operation instructing comment that is additionally stored by the additionally recording step based on the inserting length of the inserting portion that is detected by a detecting step for detecting the inserting-operation information.

# FIG.1

1

3                                              4                                                      2

| PERIPHERAL DEVICE | ENDOSCOPE MAIN BODY |

                                                                                                                    2A

STORING UNIT

5 — INSERTING-OPERATION INFORMATION COLLECTING UNIT

6 — ENDOSCOPE VIDEO OUTPUT UNIT

STANDARD INSERTING-OPERATION INFORMATION,VIDEO IMAGE,AND COMMENT

7 — COMMENT INPUT UNIT

8 — EDITING UNIT

# FIG.2

1

3                                              4                                          9                          10

| PERIPHERAL DEVICE | ENDOSCOPE MAIN BODY |

5 — INSERTING-OPERATION INFORMATION COLLECTING UNIT

6 — ENDOSCOPE VIDEO OUTPUT UNIT

ANALYZING UNIT

NOTIFYING UNIT

STORING UNIT

2 —

2A — STANDARD INSERTING-OPERATION INFORMATION,VIDEO IMAGE,AND COMMENT

# FIG.3

# FIG.4A

# FIG.4B

IMAGE
PROCESSING
UNIT

150.0

# FIG.5A

# FIG.5B

# FIG.6A

# FIG.6B

# FIG.6C

**FIG.7A**

10

11   11A   11B

LIVE   VBS

TURN FROM THIS   11C

11D

**FIG.7B**

10

11   11A   11B

LIVE   VBS

11a

**FIG.7C**

11a   20b   22b   20c

UP

21b
21a

22a
20a

DOWN

**FIG.7D**

ANGLE FOR
INSTRUCTING
INSERTING-
OPERATION
(21b,22b)

CURRENT
INSERTING
LENGTH

INSERTING
LENGTH OF
INSERTING
PORTION

# FIG.8

```
                    ┌──────────┐
                    │  START   │
                    └──────────┘
                          │
                          ▼
        ┌─────────────────────────────────────────┐  S1
        │     CALL DATA STORED ON TIME-SERIES      │
        └─────────────────────────────────────────┘
                          │
                          ▼
        ┌─────────────────────────────────────────┐  S2
        │        REARRANGE STORED DATA BASED ON    │
        │   "INSERTING LENGTH OF INSERTING PORTION"│
        │                 STORED                   │
        └─────────────────────────────────────────┘
                          │
     ┌────────────────────┤
     │                    ▼
     │  ┌─────────────────────────────────────────┐  S3
     │  │          MEASURE IN REAL TIME            │
     │  │  "INSERTING LENGTH OF INSERTING PORTION" │
     │  │         OPERATED BY OPERATOR             │
     │  └─────────────────────────────────────────┘
     │                    │
     │                    ▼
     │  ┌─────────────────────────────────────────┐
     │  │ INPUT "INSERTING LENGTH OF INSERTING PORTION"│
     │  │ TO RELATIONAL FORMULA PREPARED IN STEP S2│
     │  │ AND OBTAIN "ANGLE OF BENDING PORTION",   │  S4
     │  │    "TURN ANGLE OF INSERTING PORTION"     │
     │  │ "ENDOSCOPE IMAGE CORRELATED WITH STANDARD│
     │  │     INSERTING-OPERATION INFORMATION",    │
     │  │ AND INSTRUCTING COMMENT TO BE DISPLAYED  │
     │  └─────────────────────────────────────────┘
     │                    │
     │                    ▼
     │  ┌─────────────────────────────────────────┐  S5
     │  │   DISPLAY DATA OBTAINED IN STEP S4 ON    │
     │  │             DISPLAY DEVICE               │
     │  └─────────────────────────────────────────┘
     │                    │
     └────────────────────┘

              PERIODICALLY PROCESS
```

# FIG.9

```
1B          3                                              23
   ┌─────────────────────┐          ┌──────────────────┐
   │ PERIPHERAL DEVICE   │◄─────────│ AUTOMATIC        │
   └─────────────────────┘          │ INSERTING        │
              4 ┌──────────────────────┐  │ OPERATION        │
                │ ENDOSCOPE MAIN BODY  │◄─│ UNIT             │
                └──────────────────────┘  └──────────────────┘

   ┌──────────────────────────────────┐        ┌──────────────┐
 5 │ INSERTING-OPERATION INFORMATION  │───────►│              │
   │ COLLECTING UNIT                  │        │              │
   └──────────────────────────────────┘        │ ANALYZING    │ 9
   ┌──────────────────────────────────┐        │ UNIT         │
 6 │ ENDOSCOPE VIDEO OUTPUT UNIT      │───────►│              │
   └──────────────────────────────────┘        └──────────────┘

   ┌──────────────────────────────────┐
   │ STORING UNIT                     │
   │                                  │
 2 │    STANDARD                      │
   │    INSERTING-OPERATION           │
 2A│    INFORMATION,VIDEO             │
   │    IMAGE,AND COMMENT             │
   └──────────────────────────────────┘
```

# FIG.10

```
                          1B              14
              2                23      24A
   ┌────────────────────┐  ┌──────────┐
   │ STORED STANDARD    │  │ AUTOMATIC│
   │ INSERTING-OPERATION│─►│ INSERTING│      24B
   │ INFORMATION        │  │ OPERATION│
   │                    │  │ UNIT     │       4
 2A│                    │  └──────────┘   25A
   │                    │                  25B
   └────────────────────┘              26A
                                       26B
                                       4A
```

# FIG.11A

ANGLE OF
BENDING PORTION

TIME

# FIG.11B

INSERTING LENGTH OF
INSERTING PORTION

TIME

# FIG.11C

TURN ANGLE OF
INSERTING PORTION

TIME

# FIG.12

23

STANDARD
INSERTING-
OPERATION
INFORMATION

23b
INPUT
I/F

23a
CPU

23c
ROM

23d
RAM

23e
AMPLIFIER(#1)
I/F

23h   23f
AMPLIFIER(#2)
I/F

23i   23g
AMPLIFIER(#3)
I/F

23j

INSTRUCTION OF
ANGLE ADJUSTING
MOTOR OF BENDING PORTION

DETECTED DATA ON ANGLE OF
BENDING PORTION

INSTRUCTION OF
INSERTING-LENGTH
ADJUSTING MOTOR OF
INSERTING PORTION

DETECTED DATA ON
INSERTING-LENGTH OF
INSERTING PORTION

INSTRUCTION OF TURN ANGLE
ADJUSTING MOTOR OF
INSERTING PORTION

DETECTED DATA ON
TURN ANGLE OF
INSERTING PORTION

# FIG.13

**31**

**TRAINING ENDOSCOPE UNIT**

| PERIPHERAL DEVICE | ENDOSCOPE MAIN BODY |
|---|---|
| **3** | **4** |

**1C**

**6A**

**VIRTUAL ENDOSCOPE IMAGE DATA OUTPUT UNIT**

**32**

**EDITING AND ANALYZING UNIT**

**2**

**STORING UNIT**

**VIRTUAL IMAGE DISPLAY DEVICE**

**33**

**2A** — **STANDARD INSERTING-OPERATION INFORMATION AND VIRTUAL IMAGE**

# FIG.14

STORING UNIT

2

2A

32

EDITING AND ANALYZING UNIT

15A

15D

4

15B

31

15C

6A

VIRTUAL ENDOSCOPE IMAGE DATA OUTPUT UNIT

33

4A

VIRTUAL IMAGE DISPLAY DEVICE

VBS

VBS (SNAP SHOT)

33B

33A

33C

# FIG.15

33

LIVE

VBS

33B

33A

34

33C

# FIG.16

# FIG.17

EP 1 543 765 A1

# FIG.18

_102_

_121_  _119_

_122_  _119a_

_123a_  _121a_  _124_

_124_  _121a_

_114a_  _116_

_114a_  _115_

_114b_  _114_

_124a_

_123_

_111_

**GRAVITY DIRECTION**

# FIG.19

_122_  _123_

_121a_

_123a_

**GRAVITY DIRECTION**

# FIG.20

START IMAGE PROCESSING

DETECT BLUE OR GREEN ON OBSERVED IMAGE — S11

FIX POSITION AND MEASURE (CALCULATE) AREA — S12

ADD OPTICAL PROPERTY OF ENDOSCOPE
(ANGLE OF VIEW AND DISTORTION) — S13

THREE-DIMENSIONALLY ANALYZE
POSITIONAL RELATIONSHIP — S14

DETERMINE GRAVITY DIRECTION — S15

MATCH ROTATING DIRECTIONS OF
VBS IMAGE AND OBSERVED IMAGE — S16

END

# FIG.21

# FIG.22A

# FIG.22B

GRAVITY DIRECTION

GRAVITY DIRECTION

# FIG.23

# FIG.24

# FIG.25A

132

131a

B

A

C

IN LIQUID

GRAVITY DIRECTION

# FIG.25B

132

131a

GRAVITY
DIRECTION

IN LIQUID

EP 1 543 765 A1

# FIG.26

# FIG.27

FIG.28

# FIG.29

# FIG.30

**GRAVITY DIRECTION**

# FIG.31

*102B*

*143*

*123*

*111*

*114*    *115*

*141*

*114b*

# FIG.32

*141*

*143*

*133*

# FIG.33

141 143B 144 · · · 141 143B 144 114 111 102 115

123a · · · 123a

123 · · · 123 114b

GRAVITY
DIRECTION

# FIG.34

143B

123

# FIG.35

# FIG.36

# FIG.37

# FIG.38

# FIG.40

145A

122

123

145B

GRAVITY

# FIG.42

y

145A

x

# FIG.41

145A

121a

145B

# FIG.39

121C

145A    145B

122

# FIG.43

# FIG.44

# FIG.45

z=f PLANE

# FIG.46

*151*

*151A*

*152*

*131B*

# FIG.47

*151*

*152* *131B* *152* *151A*

*152*

*152b*

*152b*

*105*

**FIG.48A**

**FIG.48B**

EP 1 543 765 A1

FIG.49A    FIG.49B

## INTERNATIONAL SEARCH REPORT

| | International application No. |
|---|---|
| | PCT/JP03/11081 |

**A. CLASSIFICATION OF SUBJECT MATTER**
Int.Cl$^7$ A61B1/00

According to International Patent Classification (IPC) or to both national classification and IPC

**B. FIELDS SEARCHED**

Minimum documentation searched (classification system followed by classification symbols)
Int.Cl$^7$ A61B1/00-1/32, A61B6/00-6/14

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2002-200030 A (Olympus Optical Co., Ltd.), 16 July, 2002 (16.07.02), Full text; Figs. 1 to 11 | 1-10,12-18, 20,24 |
| A | Full text; Figs. 1 to 11 (Family: none) | 11,19,21-23, 25-26 |
| Y | JP 2000-135215 A (GE Yokogawa Medical Systems, Ltd.), 16 May, 2000 (16.05.00), Full text; Figs. 1 to 8 | 1-10,12-18, 20,24 |
| A | Full text; Figs. 1 to 8 (Family: none) | 11,19,21-23, 25-26 |
| Y | JP 6-304127 A (Olympus Optical Co., Ltd.), 01 November, 1994 (01.11.94), Full text; Figs. 1 to 23 (Family: none) | 3,4,7,8,14, 15,18 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 September, 2003 (24.09.03) | 07 October, 2003 (07.10.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

| International application No. |
|---|
| PCT/JP03/11081 |

C (Continuation).  DOCUMENTS CONSIDERED TO BE RELEVANT

| Category* | Citation of document, with indication, where appropriate, of the relevant passages | Relevant to claim No. |
|---|---|---|
| A | JP 5-127100 A  (Kabushiki Kaisha Machida Seisakusho), 25 May, 1993 (25.05.93), Full text; Figs. 1 to 6 & US 5280781 A | 1-26 |
| A | JP 8-542 A  (Olympus Optical Co., Ltd.), 09 January, 1996 (09.01.96), Full text; Figs. 1 to 93 & US 5840024 A          & US 6059718 A | 1-26 |
| P,A | JP 2002-345725 A  (Olympus Optical Co., Ltd.), 03 December 2002 (03.12.02), Full text; Figs. 1 to 11 (Family: none) | 1-26 |

Form PCT/ISA/210 (continuation of second sheet) (July 1998)